# EUROPEAN PATENT SPECIFICATION

(11) **EP 3 164 502 B1**
(45) Date of publication and mention of the grant of the patent: **05.12.2018**
(21) Application number: 15735900.1
(22) Date of filing: 01.07.2015
(51) Int. Cl.: C12Q 1/68

(54) **METHOD FOR DETECTING H2O2 RESISTANCE IN CRUSTACEANS**
VERFAHREN ZUR DETEKTION VON H2O2-WIDERSTAND IN SCHALENTIEREN
MÉTHODE DE DÉTECTION D'UNE RÉSISTANCE AU H2O2 CHEZ LE CRUSTACÉ

(30) Priority: 02.07.2014 US 201462019911 P; 22.12.2014 NO 20141552
(43) Date of publication of application: 10.05.2017
(73) Proprietor: PATOGEN AS, 6001 Ålesund (NO)
(72) Inventor: KAUR, Kiranpreet, 0670 Oslo (NO); HORSBERG, Tor Einar, 1279 Oslo (NO); HELGESEN, Kari Marie Olli, 0591 Oslo (NO)
(74) Representative: Onsagers AS
(86) International application number: PCT/EP2015/064972
(87) International publication number: WO 2016/001295

(56) References cited:
- WO-A1-00/57704
- Treasurer, J. W., Wadsworth, S., Grant, A.: "Resistance of sea lice, Lepeophtheirus salmonis (Kroyer), to hydrogen peroxide on farmed Atlantic salmon, Salmo salar L.", Aquaculture Research, 2000 , vol. 31 24 December 2000 (2000-12-24), XP002744099, Retrieved from the Internet: URL:http://onlinelibrary.wiley.com/doi/10. 1046/j.1365-2109.2000.00517.x/epdf [retrieved on 2015-09-07]
- Helgesen, K. O., et al: "First report of reduced sensitivity towards hydrogen peroxide found in the salmon louse Lepeophtheirus salmonis in Norway", Aquaculture Reports , 7 February 2015 (2015-02-07), XP002744100, Retrieved from the Internet: URL:http://ac.els-cdn.com/S235251341500003 4/1-s2.0-S2352513415000034-main.pdf?_tid=6 7d2d0aa-5306-11e5-ad74-00000aab0f26&acdnat =1441372448_ded6834f95010b0214c19a53066593 e1 [retrieved on 2015-09-07]
- DATABASE EMBL [Online] EBI; 2 October 2011 (2011-10-02), Leong , J. S. et al: XP002744101, Database accession no. jp322479
- DATABASE EMBL [Online] ebi; 29 March 2009 (2009-03-29), Yasuike, M. et al: XP002744102, Database accession no. bt081149
- DATABASE EMBL [Online] EBI; 30 July 2008 (2008-07-30), Koop, B., F.: XP002744103, Database accession no. fk885979
- DATABASE EMBL [Online] EBI; 1 November 2007 (2007-11-01), Lie, K., K. et al: XP002744104, Database accession no. ex733889

## Description

### Field of the invention

The present invention relates to a method for detection of elevated levels of catalase in crustaceans, such as *Lepeophtheirus salmonis,* related to resistance toward hydrogen peroxide (H₂O₂), and oligonucleotide sequences and kits useful in the method of the present invention. The present invention furthermore provides kits and reagents useful for the detection of elevated levels of catalase in *Lepeophtheirus salmonis,* related to resistance toward hydrogen peroxide (H₂O₂).

### Background of the invention

Sea lice are naturally occurring marine ectoparasites that attach to the skin and feed on the mucus, blood and surface tissues of salmon and other species of fish. Sea lice (*Lepeophtheirus salmonis* and *Caligus* spp.) are the major pathogens affecting global salmon farming industry and have a significant impact in many areas. The annual loss has recently been estimated to €300 million (Costello M. J. (2009), The global economic cost of sea lice to the salmonid farming industry. Journal of Fish Diseases. 32. 115-118) and the aquaculture industry relays heavily on a few chemotherapeutants for lice control. Emerging resistance development to these drugs increase the necessity to develop new treatment methods (biological, prophylactic and drugs) and tools to avoid increased loss due to sea lice and to ensure a sustainable salmon farming industry in the future. Control measures have relied upon a limited number of chemotherapeutants since the 1970s. Parasite resistance and reduced efficacy have now been reported for the majority of these compounds (Sevatdal S., Copley L., Wallace C., Jackson D., Horsberg T.E. (2005). Monitoring of the sensitivity of sea lice (Lepeophtheirus salmonis) to pyrethroids in Norway, Ireland and Scotland using bioassays and probit modeling). Aquaculture 244. 19-27). A successful integrated louse-management strategy requires free access to a range of effective, chemically unrelated active ingredients deployed according to current best practice. Over-reliance on a limited number of products will lead, inevitably to resistance, which is difficult to counter.

Although various chemotherapeutants have been in use in the aquaculture industry for more than 30 years, it is only during the last 15 years that such use has been part of some kind of integrated pest management (IPM) system. Management practices include coordinated salmon production within a defined area, use of single year class of fish, limited production period, fallowing, coordinated restocking, use of wrasse, synchronized treatments during the winter and targeting female lice to reduce the impact of settlement during the spring (Pike A., Wadsworth S. L., (2000), Sea Lice: A review. Advances in Parasitology. Academic Press. 44. 232-337).

Further tools are required to progress towards a true IPM-system common to other forms of food production. One key to succeeding with an IPM is to develop tools for the management of resistance to the medicines in use (Brook K. (2009). Considerations in developing an integrated pest management program for control of sea lice on farmed salmon in Pacific Canada. Journal of Fish Diseases. 32. 59-73). So far, drug resistance in sea lice has been detected by various types of bioassays as a significant increase in EC50/LC50. Although the bioassays can detect any type of resistance to a given drug, such methods are not very accurate or sensitive.

Hydrogen peroxide has been demonstrated to be the least harmful to non-target organisms, highly effective against certain stages of louse development and most environmentally responsible (Burridge, L. 2013. A review of potential environmental risks associated with the use of pesticides to treat Atlantic salmon against infestations of sea lice in southwest New Brunswick, Canada. DFO Can. Sci. Advis. Sec. Res. Doc. 2013/050. iv + 25 p). Interox® Paramove® 50 is a commercially available hydrogen peroxide product for the treatment of sea lice. It contains 50% hydrogen peroxide.

Hydrogen peroxide is only efficacious on post-chalimus stages of sea lice; it must be used in conjunction with other pest management techniques to maximize treatment benefits. There are conflicting results regarding viability of sea lice post treatment as well as the ability of lice re-infection. Because there are no techniques currently developed to remove sea lice from a tarp treatment, timing of treatment to target post-chalimus stage lice is essential. Recent studies of egg viability and nauplii survival post-treatment do indicate that nauplii survival reaches 0 within days of hatch.

Hydrogen peroxide breaks down readily into water and is considered the anti-louse treatment with least environmental risk. Recent studies comparing the effects of Interox® Paramove® 50, Salmosan®, and AlphaMax® on American lobster, mysid shrimp and sand shrimp showed that Interox® Paramove® 50 had the least impact on these non-target organisms. Unlike other anti-louse chemotherapeutants, hydrogen peroxide does not have a withdrawal period.

Hydrogen peroxide is believed to be available for use in all major salmon farming countries. It was a common louse treatment in the 1990's but was subsequently replaced by in-feed louse treatments and other bath treatments until a recent resurgence. In 2009, the use of H2O2 in aquaculture in Norway was 308,000 Kg, in 2010, 3,071,000 kg and in 2011, 1,927,000 kg.

Treatments with hydrogen peroxide are rarely fully effective and from 85% to 100% of the mobile stages may be removed (Thomassen J.M. (1993) Hydrogen peroxide as a delousing agent for Atlantic salmon. In: Pathogens in Wild and Farmed Fish: Sea Lice (ed. by G.A. Boxshall & D. Defaye), pp. 290-295. Ellis Horwood, Chichester). Hydrogen peroxide may induce mechanical paralysis caused when bubbles form in the body (Thomassen J.M. (1993) Hydrogen peroxide as a delousing agent for Atlantic salmon. In: Pathogens nf Wild and Farmed Fish: Sea Lice (ed. by G.A. Boxshall & D. Defaye), pp. 290-295. Ellis Horwood, Chichester), and these gas bubbles in the haemolymph detach the lice and cause the lice to float to the water surface (Bruno D.W. & Raynard R. (1994) Studies on the use of peroxide as a method for the control of sea lice on Atlantic salmon. Aquaculture International 2, 10-18). Many sea lice subsequently recover from treatment (Johnson S.C., Constible J.M. & Richard (1993). Laboratory investigations of the efficacy of hydrogen peroxide against the salmon louse Lepeophtheirus salmonis and its toxicological effects on Atlantic salmon Salmo salar and chinook salmon Oncorhynchus tshawytscha. Diseases of Aquatic Organisms 17, 197-204, Bruno D.W. & Raynard R. (1994) Studies on the use of peroxide as a method for the control of sea lice on Atlantic salmon. Aquaculture International 2, 10-18). It has been suggested that lice could therefore resettle on salmon (Hodneland K., Nylund A., Nisen F. & Midttun B. (1993). The effect of Nuvan, azamethiphos and hydrogen peroxide on salmon lice (Lepeophtheirus salmonis). Bulletin of the European Association of Fish Pathologists 13, 203-206.), but this was not observed in farm treatments (Treasurer J. W. & Grant A. (1997) The efficacy of hydrogen peroxide for the treatment of farmed Atlantic salmon, Salmo salar L., infested with sea lice (Copepoda: Caligidae). Aquaculture 148, 265-275).

Hydrogen peroxide (H₂O₂) is the simplest peroxide (a compound with an oxygen-oxygen single bond). It is also a strong oxidizer. Hydrogen peroxide is a clear liquid, slightly more viscous than water. In dilute solution, it appears colorless. Due to its oxidizing properties, hydrogen peroxide is often used as a bleach or cleaning agent. The oxidizing capacity of hydrogen peroxide is so strong that it is considered a highly reactive oxygen species. Concentrated hydrogen peroxide, or 'high-test peroxide', is therefore used as a propellant in rocketry (Hill, C. N. (2001). A Vertical Empire: The History of the UK Rocket and Space Program, 1950-1971. Imperial College Press. ISBN 978-1-86094-268-6).Organisms also naturally produce hydrogen peroxide as a by-product of oxidative metabolism. Consequently, nearly all living things (specifically, all obligate and facultative aerobes) possess enzymes known as catalase peroxidases, which harmlessly and catalytically decompose low concentrations of hydrogen peroxide to water and oxygen.

Two theories have been proposed to explain the therapeutic effects of hydrogen peroxide. The first is that bactericidal action is through the formation of hydroxyl radicals and its effect on DNA (Imlay J. A. (1987). The mechanisms of toxicity of hydrogen peroxide. PhD Thesis, University of California, Berkeley). The second, proposed to explain toxicity to protistans and monogeneans, is the liberation of molecular oxygen as a result of catalase action (Schaperclaus W., Kulow H. & Screkenbach K., eds. (1979) Fishkrankheiten, 4th edn. Academie-Verlag. Berlin).

Resistance of insects to pesticides develops through genetic selection of individuals (Soderlund D.M. & Bloomquist J.R. (1990) Molecular mechanisms of insecticide resistance. In: Pesticide Resistance in Arthropods (eds by R.T. Roush & B.E.Tabashnik), pp. 58-96. Chapman & Hall, London) and, in lice, this may be selection for individuals with cuticle that provides a barrier to penetration by hydrogen peroxide or the presence of detoxifying enzymes such as catalase, glutathione reductase, glutathione synthetase, superoxide dismutase, and glucose-6-phosphate dehydrogenase. An alternative explanation could be prior induction as reported for *Aeromonas salmonicida* pre-exposed to low concentrations of hydrogen peroxide (Barnes, Bowden, Horne & Ellis 1999 Barnes A.C., Balebona M.C., Home M.T. & Ellis A.E. (1999a) Superoxide dismutase and catalase in Photobacterium damselae subsp. piscicida and their roles in resistance to reactive oxygen species. Microbiology 145, 483-494 &(Barnes A.C., Bowden T.J., Horne M.T. & Ellis A.E. (1999b) Peroxide-inducible catalase in Aeromonas salmonicida subsp. salmonicida protects against exogenous hydrogen peroxide and killing by activated rainbow trout, Oncorhynchus mykiss, L., macrophages. Microbial Pathogenesis 26, 149-158). These bacteria had catalase activity 20-fold higher when subsequently exposed to higher concentrations than in un-induced cultures.

Catalase is a common enzyme found in nearly all living organisms exposed to oxygen. It catalyzes the decomposition of hydrogen peroxide to water and oxygen (Chelikani P, Fita I, Loewen PC (January 2004). "Diversity of structures and properties among catalases". Cell. Mol. Life Sci. 61 (2): 192-208. doi:10.1007/s00018-003-3206-5. PMID 14745498). It is a very important enzyme in protecting the cell from oxidative damage by reactive oxygen species (ROS). Likewise, catalase has one of the highest turnover numbers of all enzymes; one catalase molecule can convert millions of molecules of hydrogen peroxide to water and oxygen each second (Goodsell DS (2004-09-01). "Catalase". Molecule of the Month. RCSB Protein Data Bank. Retrieved 2007-02-11).

Orr et al., 1992, Archieves of biochemistry and biophysics 297:1, 35-41 reports of a study considering whether overexpression of catalase activity above the novel level prolongs the life span and provides enhanced protection against oxidative stress in *Drosophila melanogaster.* Similar findings are discussed by Mockett et al., 2003, Free radical and Biology and Medicine, 34:2, 207-2017.

Sanford et al. 1989, Journ. Of bacteriology, 171:3, 1492-1995 reports that *Dictyostelium discoideum* with catalase deficiency had an increased sensitivity to hydrogenperoxide.

In Vattanaviboon et al. 2001, FEMS microbiology Letters, 221:89-95 and Vattanabivoon and Mongkolsuk (2001),FEMS Microbiology Letters, 200: 111-116 the resistance against hydrogen peroxide in a bacteria (*Vibrio harveyi*) and is discussed.

The development of resistance by sea lice to medicines and its management is one of the main concerns in sea lice control, particularly when the range of medicines is limited. Resistance of sea lice to pesticides, particularly organophosphates and pyrethroids, is well established. However, resistance towards hydrogen peroxide (H₂O₂) has only been reported ones (J W Treasurer, S Wadsworth & A Grant. (2000) Resistance of sea lice, Lepeophtheirus salmonis (Kroyer), to hydrogen peroxide on farmed Atlantic salmon, Salmo salar L. Aquaculture Research, 31, 855-860). However, in the autumn of 2013, reduced treatment efficacy was reported from salmon farms in the north of Norway. The cause or mechanisms behind such resistance in salmon lice against hydrogen peroxide has not been known previously.

Up to date, resistance in sea lice towards hydrogen peroxide (H₂O₂) has not been acknowledged as a major concern in the aquaculture industry, except for the report by Orr et al. in 1992, *supra.* No documentation related to the mechanism of such resistance have existed. Also, no specific mechanism on the molecular level has been associated with resistance towards hydrogen peroxide (H₂O₂) in any parasites, including sea lice.

Efficient and sensitive methods for diagnosing resistance are crucial in order to manage and control drug resistance. Early detection of reduced sensitivity to a chemical can enable effective countermeasures to be enforced at a time point when these have a greater probability of being effective. Therefore, accurate and speedy identification of hydrogen peroxide (H₂O₂) resistant sea lice is crucial. Detection of hydrogen peroxide (H₂O₂) resistance prior to treatment, and the use of such analyses after treatment to evaluate treatment efficacy constitutes an important determinant for the integrated pest management (IPM) in the aquaculture industry.

### Summary of invention

The present invention is based on the surprising finding that resistance towards hydrogen peroxide (H₂O₂) commonly used to combat sea lice infestation is linked to the expression level of the enzyme catalase. The present inventors have identified the gene encoding a catalase enzyme, and its relevance in the development of hydrogen peroxide resistance in sea lice. Furthermore, the present inventors have developed Real-Time PCR-assays to quantify the expression of this catalase encoding gene, and correlated this expression to the resistance toward hydrogen peroxide (H₂O₂) in sea lice. More particularly, the present invention is based on the quantification of the expression of the gene encoding the catalase of the sea lice (*Lepeophtheirus salmonis*) shown to be involved in the resistance towards hydrogen peroxide (H₂O₂) -based chemotherapy.

Thus, according to one embodiment, and in vitro method is provided for the detection of hydrogen peroxide resistance in one or more crustaceans comprising the steps of determining the catalase activity in the crustaceans to be analyzed.

According to one embodiment, an in vitro method is provided for detection of hydrogen peroxide (H₂O₂) resistance in one or more crustaceans comprising the steps of quantifying the expression of a catalase gene of the crustaceans to be analyzed. According to one embodiment, said catalase gene has a sequence as depicted in SEQ ID No. 1, or variants or fragments thereof being at least 70 % identical with SEQ ID No. 1.

The crustacean may be one or more copepods, e.g. belonging to the family Caligidae. According to one embodiment, the copepod is selected from the group consisting of *Lepeophtheirus salmonis, Caligus clemensei, Caligus elongatus,* and *Caligus rogercresseyi.*

According to one embodiment, said catalase gene origins from *Lepeophtheirus salmonis* and has a sequence as depicted in SEQ ID No. 1, or variants or fragments thereof being at least 70 % identical with SEQ ID No. 1, such as 80% identical with SEQ ID No.1.

According to another embodiment, said catalase gene origins from *Lepeophtheirus salmonis* and has a sequence as depicted in SEQ ID No. 1, or variants or fragments thereof being at least 80 % identical with SEQ ID No. 1.

According to another embodiment, said catalase gene origins from *Lepeophtheirus salmonis* and has a sequence as depicted in SEQ ID No. 1, or variants or fragments thereof being at least 90 % identical with SEQ ID No. 1

According to one embodiment, said catalase gene origins from *Caligus clemensei* and has a sequence as depicted in SEQ ID No. 13, or variants or fragments thereof being at least 70 % identical with SEQ ID No. 13, such as 80% identical with SEQ ID No. 13.

According to another embodiment, said catalase gene origins from *Caligus clemensei* and has a sequence as depicted in SEQ ID No. 13, or variants or fragments thereof being at least 90 % identical with SEQ ID No. 13

According to yet another embodiment, said catalase gene origins from *Caligus rogercresseiy* has a sequence as depicted in SEQ ID No. 14, or variants or fragments thereof being at least 70 % identical with SEQ ID No. 14, such as 80% identical with SEQ ID No. 14. According to yet another embodiment, said catalase gene origins from *Caligus rogercresseiy* has a sequence as depicted in SEQ ID No. 14, or variants or fragments thereof being at least 90 % identical with SEQ ID No. 14

According to another embodiment, the present method comprise the steps of detecting increased RNA-expression levels associated with hydrogen peroxide (H₂O₂) resistance in a crustacean to be analyzed, wherein said crustacean is resistant to hydrogen peroxide (H₂O₂) resistance if having elevated levels of catalase RNA-expression.

According to another aspect of the present invention, a method is provided comprising the steps of:
a) collecting sea lice from infested fish or water samples;
b) isolating genomic material from the any life stage of collected sea lice;
c) determining the expression level of a catalase gene.

According to one embodiment, the determination of expression level in c) is determined by measuring the catalase RNA-expression level, wherein said sea lice is resistant to hydrogen peroxide (H₂O₂) if the catalase RNA-expression level is elevated.

According to another embodiment of the present method, said step c) is performed using a primer selected from the group consisting of SEQ ID No.'s 2, 3, 4, 5, 7, 8, 10 and 11. According to another embodiment of the present disclosure said step c) is performed using a primer selected from the group consisting of SEQ ID No.'s 15, 16, 18 and 19.

According to another embodiment of the present method, said step c) is performed using at least one probe selected from the group consisting of SEQ ID No.'s 6, 9 and 12. According to another embodiment of the prsent dsclosure, said step c) is performed using at least one probe selected from the group consisting of SEQ ID No.'s 17 and 20.

According to another embodiment of the present method, said step c) comprises nucleic acid amplification.

According to another embodiment of the present method, the nucleic acid amplification is performed using polymerase chain reaction.

According to another embodiment of the present method, said step c) is performed by contacting the genomic material of the sea lice to be analyzed with a detection reagent, and determining the expression level of catalase mRNA.

According to the above embodiments, the determination of hydrogen peroxide (H₂O₂) resistance takes the advantage of determining the expression level of the gene encoding catalase in the crustaceans to be analyzed. Hydrogen peroxide (H₂O₂) resistance may also be determined by analyzing the level of catalase activity, as increased expression level of the catalase gene may be shown as an increased catalase activity level in the hydrogen peroxide resistant crustaceans.

According to another embodiment, the present method comprises the steps of detecting increased catalase activity in sea lice. Detection of catalase activity in sea lice may e.g. be performed using commercially available kits for measuring catalase enzyme activity, wherein said crustacean is resistant to hydrogen peroxide (H₂O₂) resistance if having elevated levels of catalase activity as a result of increased catalase gene expression. According to another aspect of the present invention, a method is provided comprising the steps of:
a) collecting sea lice from infested fish or water samples;
b) homogenization of sea lice tissue samples from any life stage of collected sea lice;
c) determining the catalase activity, wherein said sea lice is resistant to hydrogen peroxide (H₂O₂) if the catalase activity is elevated.

According to one embodiment, the determining of catalase activity step c) is performed by collecting a sample isolated from the crustaceans to be analyzed with a pre-determined amount of hydrogen peroxide, and then with horseradish peroxidase and a chromophore, resulting in the formation of a chroma signal, and wherein the decomposition of hydrogen peroxide is proportional with the level of catalase in the sample. According to one embodiment, the formation of said chroma signal is measured colorometrically.

The present disclosure also provides for an isolated oligonucleotide sequence encoding a catalase of which the expression level is associated with hydrogen peroxide (H₂O₂) resistance. According to one embodiment, said nucleic acid sequence has the sequence as depicted in SEQ ID No. 1.

According to yet another embodiment, the oligonucleotide sequence of the present invention is identical or has at least 70% sequence identity with the SEQ ID No. 1 SEQ ID No. 13 or SEQ ID No. 14 and sequences having at least 70% sequence identity with the SEQ ID No. 1, SEQ ID No. 13 or SEQ ID No. 14, respectively, or a fragment thereof, and complementary sequences of SEQ ID No 1 and fragments thereof, provided that said sequence are not identical with the sequence depicted in SEQ ID No. 21 or SEQ ID No. 22.
SEQ ID No. 21 correspond to a sequence published the 27^{th} March 2009 assigned the Genbank accession number BT081149 (mRNA sequence encoding catalase from *Caligus clemensi*).
SEQ ID No. 22 correspond to a sequence published the 11^{th} June 2013 assigned the Genbank accession number KF233999 (mRNA sequence encoding catalase from *Octopus vulgaris*).

The present invention furthermore provides for oligonucleotide sequences, such as primers and probes, useful in the detection of catalase gene expression in crustaceans according to the present invention. The said oligonucleotide sequences may be selected from the group consisting of SEQ ID No.'s 2-12, and fragments and variants thereof, having at least 70 % sequence identity with the said sequences SEQ ID No.'s 2-12, and complementary sequences thereof.

Furthermore, the present invention provides a kit for detection of hydrogen peroxide (H₂O₂) resistance in crustaceans comprising at least one oligonucleotide according to the present invention as defined in the appended claims.

Finally, the present invention provides the use of one or more isolated oligonucleotide sequence(s) comprising at least 8 contiguous nucleotides of the sequence SEQ ID No. 1 or a complementary oligonucleotide thereof for the determination of hydrogen peroxide in crustaceans as defined in the appended claims.

The present invention and its various embodiments will be described in more detail in the following description and defined in the appended claims.

### Figures

**Figure 1** shows the Catalase gene sequence from the *Lepeophtheirus salmonis* (SEQ ID No. 1).
**Figure 2** shows the difference in catalase expression is significant between the H2O2-resistant strain (labelled H2O2), and the other two strains when using the calculated ratio value for catalase / elongation factor in each sample.
**Figure 3** shows the distribution of normalized Ct-values in the whole dataset. A biphasic distribution with one peak at Ct ∼16 and one at Ct ∼20 can be observed.
**Figure 4a** shows the distribution of normalized Ct-values within treatments.
**Figure 4b** shows the distribution of normalized Ct-values within treatments and survival status.
**Figure 5** shows the Wilcoxon test of rank sums for the difference in normalized Ct-values between immobilized and alive parasites for both treatment groups. For both groups, the difference is highly significant (p<0.0001).
**Figure 6a** shows the Wilcoxon test of rank sums for the difference in normalized Ct-values between immobilized ("immob") parasites in the two treatment groups, and a corresponding test for live ("lev") parasites.
**Figure 6b**. Estimated probability of immobilization (red line) with 95% confidence intervals (dotted lines) as a function of normalized Ct values in the experiments exposing parasites alone (Parasitt) and parasites on fish (Fisk) to H₂O₂. Vertical symbols along y = 0 and Y = 1 denote Ct values of the live and immobilized lice, respectively.
**Figure 7** shows the output from the ANOVA analysis of catalase activity (U/ug protein) versus the strain of salmon lice.
**Figure 8** shows the output from the ANOVA analysis of expression of the catalase gene (normalized Ct-values) versus the strain of salmon lice.
**Figure 9** shows the output from the ANOVA analysis of normalized Ct-values versus the strain of salmon lice.
**Figure 10** shows the output from the Tukey-Kramer HSD test of normalized Ct-values versus the strain of salmon lice.
**Figure 11** shows the clustering of strains based on the normalized Ct-values.

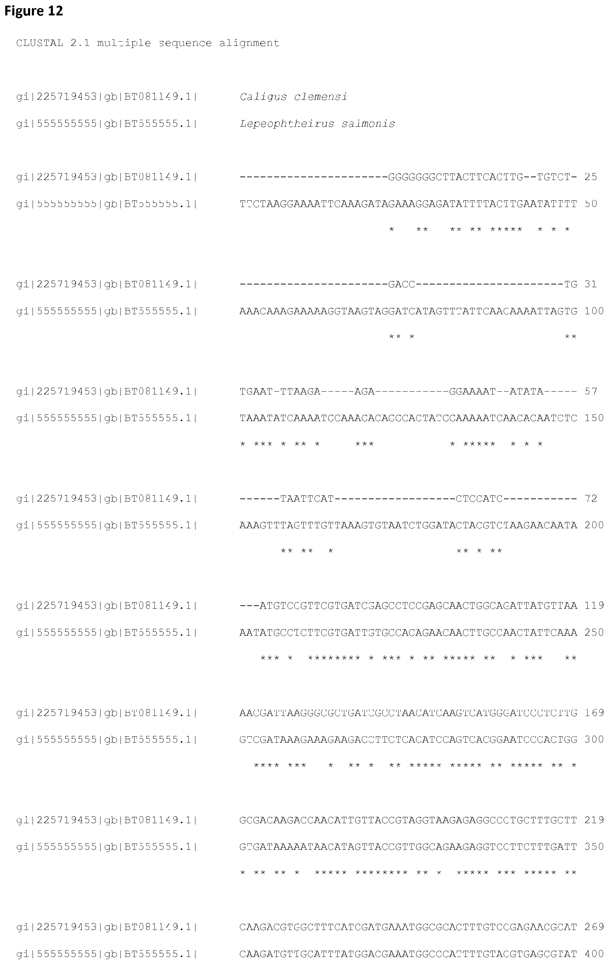

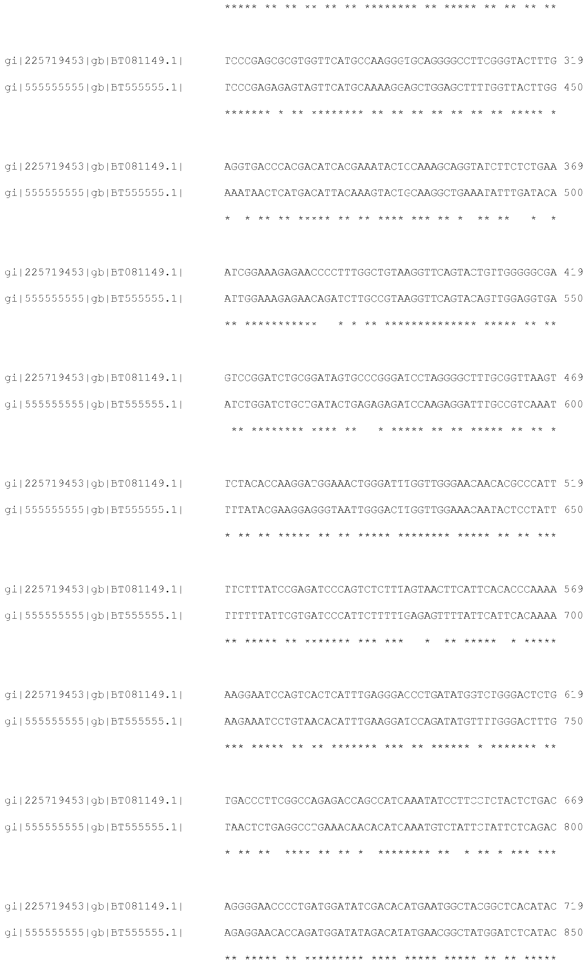

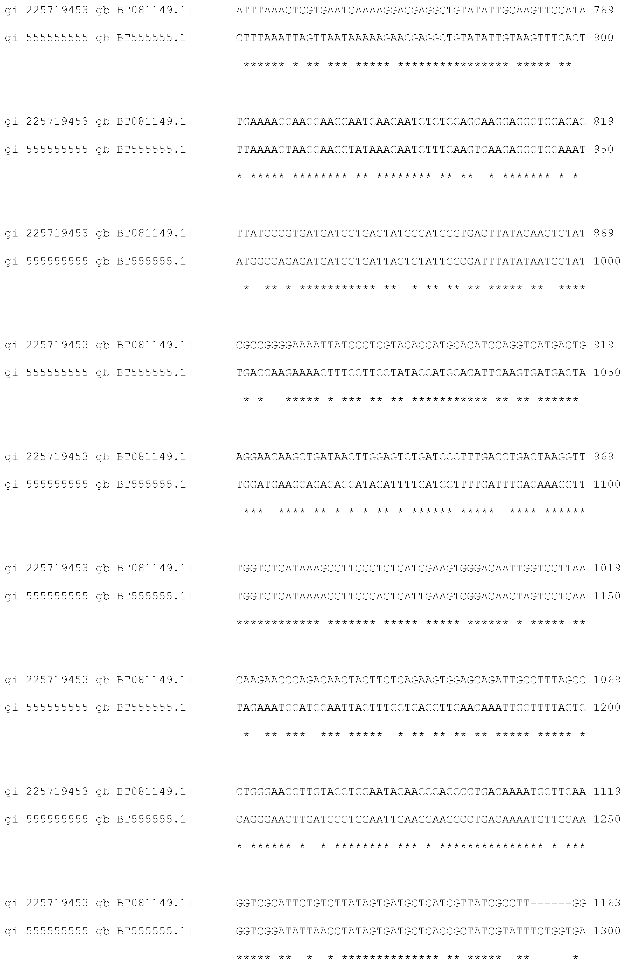

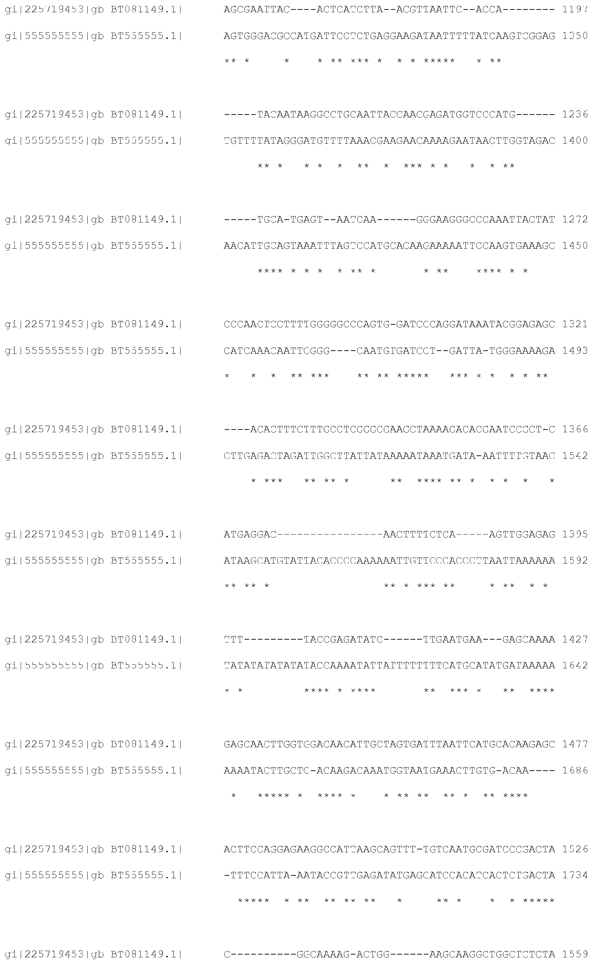

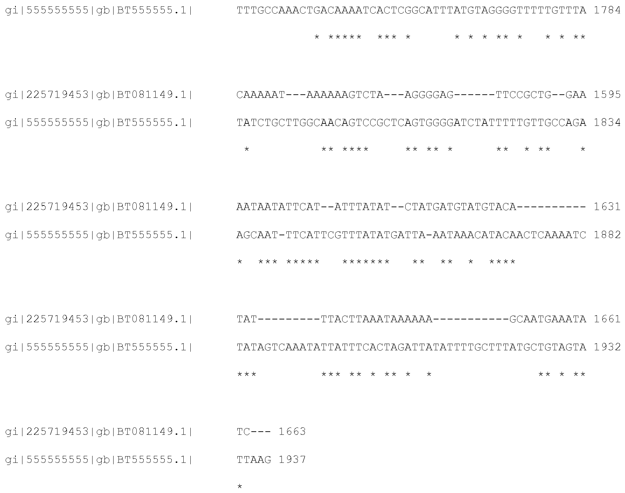

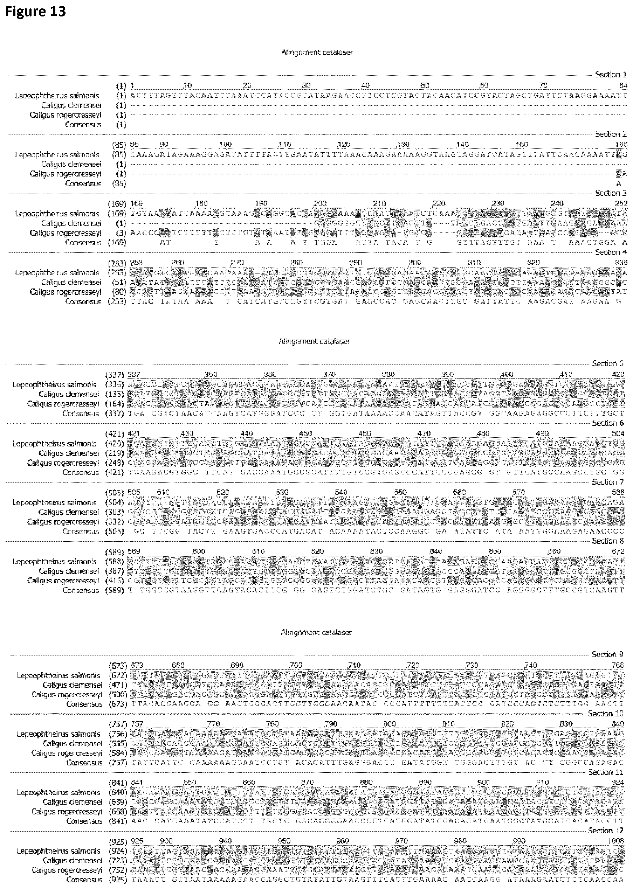

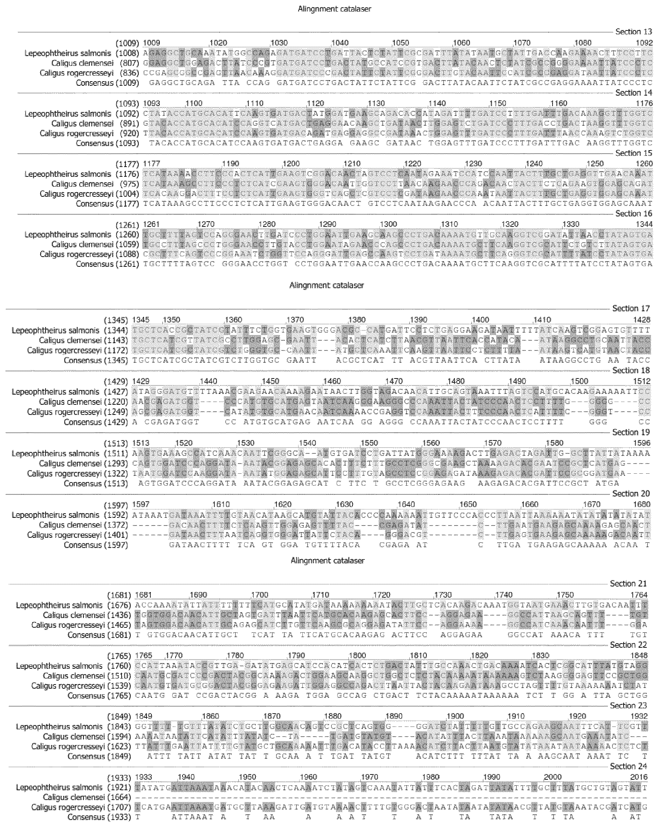

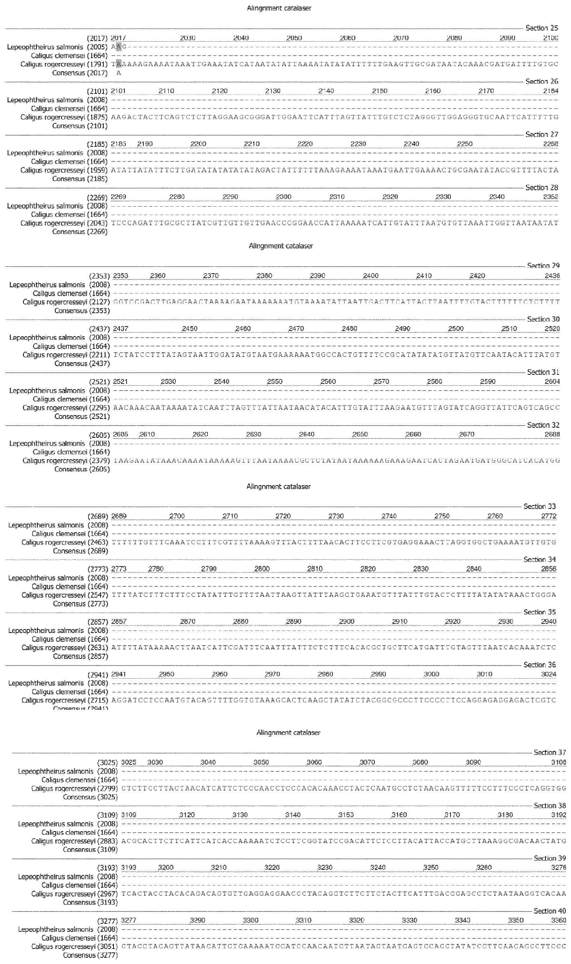

**Figure 12** shows the alignment between the catalase genome sequence from *Lepeophtheirus salmonis, Caligus rogercresseyi* and *Caligus clemensi.*

### Detailed description of the invention

The present invention provides an in vitro method for determination of hydrogen peroxide (H₂O₂) resistance in crustaceans, including copepods, in particular *Lepeophtheirus salmonis,* based on the surprising findings that the expression level of catalase gene is linked with resistance against hydrogen peroxide.

The increased level of catalase gene expression results in increased catalase activity in the hydrogen peroxide resistant crustacean. The expression "catalase activity" as used in the present invention is thus to be read in the context of being a result of increased catalase gene expression, irrespective of the method used to determine said increased catalase gene expression and/or catalase activity.

Although the experimental data linking the catalase gene expression level and hydrogen peroxide resistance was identified in the sea lice species *Lepeophtheirus salmonis,* the skilled person will acknowledge, based on the teaching herein, that the present method and the present oligonucleotides may be used to determine hydrogen peroxide resistance in crustaceans, in particular copepods, in particular copepods belonging to the family Caligidae. In particular, it is to be understood that the present method and the present oligonucleotides may be used to determine hydrogen peroxide resistance in copepods affecting farmed fish, such as e.g. fish belonging to the family *Salmonidae.* According to one embodiment disclosed herein, the present method and present oligonucleotides are useful for detection of hydrogen peroxide resistance in copepod selected from the group consisting of *Lepeophteirus salmonis, Caligus clemensei, Caligus elongatus,* and *Caligus rogercresseyi.*

Throughout the disclosure, the term "sea louse" or "sea lice" is to be understood to mean one or more copepod belonging to the family Caligidae. In the experimental data provided in the present disclosure, "sea lice" or "sea louse" refer to the species *Lepeophtheirus salmonis.* By establishing a bioassay to differentiate sensitive vs resistant populations of sea lice, characterizing several lice strains with respect to sensitivity, documenting that the resistance is inheritable, measuring catalase enzyme activity, sequencing the catalase gene and establishing Real-Time PCR-assays, the present inventors have found that the hydrogen peroxide resistance is linked with the level of expression of the catalase gene.

In particular, the present disclosure provides a method wherein at least one hydrogen peroxide resistant sea louse is determined by determining the catalase expression level of the sea lice to be tested.

The sequences provided in table 3 below may according to one embodiment be used to determine hydrogen peroxide resistant sea lice of the species *Lepeophtheirus salmonis.* The present inventors have furthermore provided sequences that may be used according to the present method to determine hydrogen peroxide resistant sea lice of the species *Caligus clemensei* and *Caligus rogercresseyi,* i.e. having the following sequences:

| **Species** | **Forward primer** | **Revers primer** | **Probe** |
|---|---|---|---|
| | CR-ST-F | CR-ST-R | CR-ST-P |
| *Caligus rogercress eyi* | AAGAGGAATCCTGTGACACACTTG (SEQ ID No. 15) | CTGGTCGGAGTGTGACAAAGTC (SEQ ID No. 16) | ACCCCGACATGGTATG (SEQ ID No. 17) |
| | Cali-Cata-F | Cali-Cata-R | Cali-Cata-P |
| *Caligus clemensei* | GCGCACTTTGTCCGAGAAC (SEQ ID No. 18) | CTGCACCCTTGGCATGAAC (SEQ ID No. 19) | CATTCCCGAGCGCGT (SEQ ID No. 20) |

According to one embodiment, the present invention provides a method for characterizing the hydrogen peroxidase sensitivity of one or more sea lice, comprising the steps of
a) determining in a biological sample comprising DNA or RNA of one or more sea lice the expression level of the catalase gene according to SEQ ID No. 1 or a variant or fragment thereof;
b) comparing the determined catalase expression level to a control standard or the expression of the catalase gene, or a variant or fragment thereof, in a control sample;
c) determining whether the difference in expression level in the sample as compared to the control standard or the expression of catalase in a control sample; and
d) characterizing the sea lice, wherein the sea lice to be tested has a higher expression level compared with the control standard or the control sample.

According to one embodiment, said method includes the steps of
a) collecting one or more sea lice from infested fish or water samples;
b) isolating DNA or RNA from the collected sea lice of step a)
c) providing a pair of PCR primers specific for the catalase gene of SEQ ID No. 1;
d) performing PCR on the nucleic acid of step b) using the pair of PCR primers of step c);
e) determining the expression level of the catalase gene by comparing the level of catalase expression with the level of control standard or a catalase gene expression sample control.

According to one embodiment, the level of catalase gene expression control is the level of catalase expression in H₂O₂ sensitive sea lice.

According to another embodiment, a H₂O₂ resistant sea louse is a sea louse having a catalase expression level that are significantly higher compared with the catalase expression level in a H₂O₂ sensitive sea louse.

According to another embodiment, a method is provide wherein one or more H₂O₂ resistant sea lice is determined using a primer pair selected from the group of primer pair consisting of SEQ ID no. 2 and 3, SEQ ID No. 4 and SEQ ID No. 5, SEQ ID No. 7 and SEQ ID No. 8, and SEQ ID No. 10 and SEQ ID No. 11.

According to another aspect of the present invention, the catalase expression level is measured using a catalase activity assay, e.g. wherein the method of the invention comprises the steps of:
a) providing a sample to be tested;
b) add H₂O₂ to the sample of a) to the sample of a)
c) stop the reacting of b) by adding a catalase inactivator
d) determine the remaining amounts of H₂O₂
e) determine the amount of catalase present in the sample
f) compare the results of step e) with a control sample of a H₂O₂ sensitive sea louse and determine the catalase expression level of the test sample.

Furthermore, as used herein, an "oligonucleotide sequence" or "nucleic acid sequence" is to be understood to mean an oligonucleotide sequence or a nucleic acid sequence useful in determining the expression of the catalase enzyme gene, e.g. the catalase enzyme gene depicted in SEQ ID No 1. An "oligonucleotide sequence" or "nucleic acid sequence" used to determine the expression level of catalase gene is capable of hybridize to a nucleic acid sequence with a complementary sequence, such as e.g. mRNA extracted from the copepod to be analyzed for hydrogen peroxide resistance.

The skilled person is well aware of the fact that nucleic acid molecules may be doublestranded or single-stranded, and that reference to a particular site of one strand refers, as well, to the corresponding site on a complementary strand. Thus, reference to an adenine (A), a thymine (T) (uridine (U)), a cytosine (C) or a guanine (G) at a particular site on one strand of a nucleic acid is also to be understood to define a thymine (uridine), adenine, guanine, or cytosine, respectively, at the corresponding site on a complementary strand of the nucleic acid molecule. Thus, reference may be made to either strand in order to refer to a particular position. The oligonucleotide probes and oligonucleotide primers according to the present invention may be designed to hybridize to either strand.

An "isolated nucleic acid" useful in the detection method of the present invention, i.e. such as primers and probes, as used herein is generally one that contains at least 8 nucleotides and which is capable of hybridizing a nucleic acid with a complementary sequence, and is separated from most other nucleic acids present in the natural source of the nucleic acid, and is thus substantially free of other cellular material.

### Oligonucleotide probes and oligonucleotide primers

The present invention provides oligonucleotide probes and oligonucleotide primers that may be used for determining the level of expression of the catalase gene being linked with hydrogen peroxidase resistance in a crustacean to be tested in accordance with the present invention. The determination of the expression level of a gene is widely applied in both human and veterinary diagnosis, wherein nucleic acids from e.g. pathogens present in biological samples are isolated and hybridized to one or more hybridizing probes or primers are used in order to amplify a target sequence.

One or more oligonucleotide probes may be constructed based on the teaching herein and used in hybridization based detection methods where upon the binding of the oligonucleotides to the target sequence enables determination of the level of expression of a gene present in the crustacean to be tested.

The skilled person will acknowledge that an oligonucleotide probe according to the present invention may be a fragment of DNA or RNA of variable length used herein in order to hybridize to the target sequence, e.g. single-stranded DNA or RNA. The oligonucleotide probe according to the present invention may furthermore be labeled with a molecular marker in order to easily visualize that hybridization have been achieved. Molecular markers commonly known to the skilled person may be used, e.g. a radiolabel, and more preferably, a luminescent molecule or a fluorescent molecule enabling the visualisation of the binding of the probe(s) to a target sequence.

A oligonucleotide probe according to the present invention is able to hybridize to another nucleic acid molecule, such as the single strand of DNA or RNA originating from a crustacean to be analysed, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognise as the "stringency" of the hybridization. The suitable stringency for hybridisation of a probe to target nucleic acids depends on inter alia the length of the probe and the degree of complementation, variables well known to the skilled person. A oligonucleotide probe according to the present disclosure typically comprises a nucleotide sequence which under stringent conditions hybridize to at least 8, 10, 12, 16, 20, 22, 25, 30, 40, 50 (or any other number in-between) or more consecutive nucleotides in a target nucleic acid molecule, e.g. single-stranded DNA or RNA isolated from the crustacean to be analyzed according to the present invention. According to one embodiment, the oligonucleotide probe according to the present invention comprises about 13 to 25 consecutive nucleotides. New technology like specific Locked Nucleic Acid (LNA) hybridization probes allows for the use of extremely short oligonucleotide probes (You Y.; Moreira B.G.; Behlke M.A. and Owczarzy R. (2006). "Design of LNA probes that improve mismatch discrimination". Nucleic Acids Res. 34 (8): e60. doi:10.1093/nar/gkl175. PMC 1456327. PMID 16670427.) According to one embodiment, probes are provided which are selected from the group consisting of SEQ ID No. 6, SEQ ID No. 9 and SEQ ID No. 12.

The present invention furthermore provides oligonucleotide primers useful for amplification of any given region of a nucleotide sequence. An oligonucleotide primer according to the present disclosure typically comprises a nucleotide sequence at least 8, 10, 12, 16, 20, 22, 25, 30, 40, 50 (or any other number in-between) or more consecutive nucleotides. According to one embodiment, the oligonucleotide primer according to the present invention comprises about 18 - 25 consecutive nucleotides, more preferably about 20 nucleotides.

As used herein, the term "oligonucleotide primer" is to be understood to refer to a nucleic acid sequence suitable for directing an activity to a region of a nucleic acid, e.g. for amplification of a target nucleic acid sequence by polymerase chain reaction (PCR).

The skilled person will acknowledge that an oligonucleotide primer according to the present invention may be a fragment of DNA or RNA of variable length used herein in order to determine the expression level of the target sequence, e.g. single-stranded DNA or RNA, upon alignment of the oligonucleotide probe to complementary sequence(s) of the said target sequence to be analyzed. An oligonucleotide primer according to the present invention may furthermore be labeled with a molecular marker in order to enable visualization of the results obtained. Various molecular markers or labels are available. An oligonucleotide primer according to the present invention typically comprises the appropriate number of nucleotides allowing that said primer align with the target sequence to be analyzed. It is to be understood that the oligonucleotide primer according to the present invention according to one embodiment may comprise SNP's or the complement thereof. According to one embodiment, the primers useful in order to determine hydrogen peroxide resistant sea lice may be selected from the group consisting of the primer pairs SEQ ID No. 2 and SEQ ID No. 3, SEQ ID No. 4 and SEQ ID No. 5, SEQ ID No. 7 and SEQ ID No. 8, and SEQ ID NO. 10 and SEQ ID No. 11.

Oligonucleotide probes and oligonucleotide primers according to the present invention may be synthesizes according to methods well known to the skilled person.

The present disclosure furthermore relates to isolated nucleic acid sequences and variants or fragments thereof having at least 70% identity with the nucleic acid sequences depicted in SEQ ID NO.'s 1-12 or fragments thereof. The term "% identity" is to be understood to refer to the percentage of nucleotides that two or more sequences or fragments thereof contains, that are the same. A specified percentage of nucleotides can be referred to as e.g. 70% identity, 80% identity, 85% identity, 90% identity, 95% identity, 99% identity or more (or any number in between) over a specified region when compared and aligned for maximum correspondence.

According to one embodiment, the disclosure includes oligonucleotide sequence encoding a catalase of which the expression level is associated with hydrogen peroxide resistance in sea lice, wherein said nucleic acid sequence has the sequence as depicted in SEQ ID No. 1, SEQ ID No. 13 or SEQ ID No. 14 or fragments thereof, and sequences having at least 80% sequence identity with the SEQ ID No. 1, SEQ ID No. 13 or SEQ ID No. 14, respectively, and complementary sequences of SEQ ID No 1, SEQ ID No. 13, and SEQ ID No. 14, respectively.

The skilled person will acknowledge that various means for comparing sequences are available. For example, one non-limiting example of a useful computer homology or identity program useful for determining the percent homology between sequences includes the Basic Local Alignment Search Tool (BLAST) (Altschul et al., 1990, J. of Molec. Biol., 215:403-410, "The BLAST Algorithm; Altschul et al., 1997, Nuc. Acids Res. 25:3389-3402, , Karlin and Altschul 1990, Proc. Nat'l Acad. Sci. USA, 87:2264-68; 1993, Proc. Nat'l Acad. Sci. USA 90:5873-77).

### Methods for determination of the catalase activity by measuring the expression level of catalase gene.

Based on the teaching herein, i.e. that the expression level of catalase gene are linked with hydrogen peroxide resistance in crustacean, the skilled person will acknowledge that various well known methods are available for the determination of the expression level of genes, such as the expression of catalase gene within a population of crustacean, may be applicable in the present method. For example, methods based on genome sequencing, hybridization and enzyme assay based methods are applicable for determining whether a crustacean is hydrogen peroxidase resistant in accordance with the present inventions. Various enzyme based methods are available for the skilled person for this purpose, of which a number of polymerase chain reaction (PCR) based methods are available. Oligonucleotide primers according to the present invention useful in such a method may be selected from the group consisting of SEQ ID No. 2, SEQ ID No. 3, SEQ ID No. 4, SEQ ID No. 5, SEQ ID No. 7, SEQ ID No. 8, SEQ ID NO. 10 and SEQ ID No. 11.

Several hybridization methods for detection of a nucleic acid sequence of interest, such as e.g. a gene sequence or a mRNA encoding the catalase enzyme (or one or more parts thereof) are available to the skilled person, and which may be utilized in accordance with the method of the present invention. For example, the oligonucleotides according to the present invention may be detected utilizing molecular beacon technology. According to this aspect of the present invention, oligonucleotide primers may be synthesized comprising complementary regions at each end allowing the formation of a hairpin loop, and wherein a fluorophore is attached at one end of the oligonucleotide primer, and a quenching agent is attached to the other end, and wherein fluorescence signal is produced upon binding to a target sequence.

### Isolation of sea lice genomic material

The method according to the present invention may according to one embodiment involve the isolation of a biological sample from a crustacean and measuring the level of expression of catalase in order to determine whether the crustacean is hydrogen peroxide resistant.

In order to determine whether a crustacean, such as sea lice, is hydrogen peroxide resistant in accordance with the present invention, genomic material may be isolated. Various methods for obtaining genomic material well known to the skilled person are available. The skilled person will acknowledge that any tissue (i.e. any part of the sea lice) may be used in order to extract genomic material. Furthermore, the genomic material to be analyzed according to the present invention may be obtained from sea lice of any life stages, e.g. the free swimming stages (nauplius stage I and II), the copepod stage, the pre-adult (chalimus stages 1-4), or the adult stage (adult male or adult female). According to one embodiment, tissue removed from sea lice to be tested is maintained in 70% ethanol or other conservation liquid prior to further isolation of genomic material. DNA may be extracted from the obtained tissue using commonly available DNA extraction/isolation methods, such as e.g. DNeasy DNA Tissue Kit according to the protocol of the manufacturer (http://lycofs01.lycoming.edu/∼gcat-seek/protocols/DNeasy_Blood_&_Tissue_Handbook.pdf).

### Catalase gene expression detection kits

Based on the teaching herein, the skilled person will acknowledge that, based on the identification of the link between hydrogen peroxide resistance and catalase activity in crustaceans, reagents applicable in determination of catalase activity and/or catalase gene expression level can be developed for the determination of hydrogen peroxidase resistance. The term "kit" as used herein in the context of catalase activity determination detection reagents is intended to cover reagents useful for determination of catalase activity, both in respect of determining the level of catalase gene expression or by determining the activity of catalase present in a sample to be analyzed.

For example, according to the present invention, a kit may comprise oligonucleotide probe(s) or oligonucleotide primer(s) or primer sets, arrays/microarrays of nucleic acid molecules, and beads that contain one ore more oligonucleotide probe(s), oligonucleotide primer(s) or other detection reagents useful in the method of the present invention. It is furthermore to be understood that the detection reagents in a kit according to the present invention may furthermore include other components commonly included in such kits, e.g. such as various types of biochemical reagents (buffers, DNA polymerase, ligase, deoxynucleotide triphosphates for chain extension/amplification, etc.), containers, packages, substrates to which detection reagents are attached., etc. necessary to carry the method according to the present invention. According to one embodiment of the present invention, a kit is provided which comprises the necessary reagents to carry out one or more assays in order to determine the catalase gene expression level according to the method of the present invention. A kit according to the present invention may preferably comprise one or more oligonucleotide probes that hybridize to a nucleic acid target molecule (i.e. genetic material) enabling determination of the catalase gene expression level in the material analyzed. Multiple pairs of probes may be included in the kit to simultaneously analyze for determination of catalase gene expression at the same time. The probes contained in the kit according to the present invention may according to one embodiment be immobilized on a carrier, such as e.g. an array or a bead.

According to one embodiment, a kit according to the present invention comprises oligonucleotide primer(s) and optionally further reagents useful in methods for the determination of catalase gene expression utilizing oligonucleotide primers or primer pair(s). According to one embodiment, the kit according to the present invention comprises a forward primer and a reverse primer for amplifying a region of the catalase gene. Said kit may furthermore optionally comprise further reagents (enzymes and nucleotide triphosphates) necessary for conducting PCR or real time PCR.

### Detection of catalase activity

According to the present invention, the high expression level of catalase seen in hydrogen peroxide resistance sea lice may also be determined measuring the catalase activity in sea lice to be analyzed, as increased catalase gene expression results in increased catalase activity.

Various protocols useful for measuring catalase activity are known to the skilled person and are useful in measuring catalase activity in accordance with the present invention. Many of the well known catalase assays available to the skilled person measure the catalase activity by contacting the sample to be analyzed with a predetermined amount of hydrogen peroxide, and then with horseradish peroxidase and a chromophore. Catalase present in the sample to be analyzed decomposes hydrogen peroxide according to the following equation:

2H₂O₂ → 2H₂O + O₂

The horseradish peroxidase reacts with the chromophore using the remaining hydrogen peroxide as an oxidizing agent, resulting in the formation of a chroma signal which may be measured using well known colorimetric methods. The mere catalase present in the sample, the less chroma signal is formed. Non-limiting examples of chromophores to be used according to this aspect of the present invention is 3,3',5,5'-tetramethylbenzidin, 3,3'-diaminobenzidine and 2,2'-azino-bis(3-ethylbenzothiazoline-6-sulphonic acid).

According to another embodiment of the present method, step c) of the above method is performed by detecting increased catalase activity in sea lice using commercially available kits for measuring catalase enzyme activity. Commercially available catalase assay kits are provided by e.g. Sigma-Aldrich ® (CAT100-1KT), cf. http://www.sigmaaldrich.com/catalog/product/sigma/cat100?lang=en&region=NO, Cayman Chemical Company ®, see https://www.caymanchem.com/pdfs/707002.pdf. Oxford Biochemical Research ®, see http://www.oxfordbiomed.com/catalase-assay-kit, and OxiSelectTM Catalase Activity Assay Kit from Cell Biolabs, Inc., see http://www.cellbiolabs.com/sites/default/files/STA-341-catalase-activity-assay.pdf.

### EXAMPLES

Reduced efficacy of treatments with hydrogen peroxide (H₂O₂) has been observed in Nord-Trøndelag and Nordland counties in Norway. A salmon lice strain from this area (LS H₂O₂) has been cultivated in the wet-lab at the NIVA marine research station, Solbergstrand, Drøbak. The offspring of the original strain has been subjected to bioassays with hydrogen peroxide using the protocol of Sevatdal S and Horsberg TE (2003), "Determination of reduced sensitivity in sea lice (Lepeophtheirus salmonis Kroyer) against the pyrethroid deltamethrin using bioassays and probit modelling", Aquaculture 218, 21-31, adjusted for hydrogen peroxide using an exposure period of 30 minutes. The effect of the assay was determined immediately after exposure, as previous tests have demonstrated that around 80 % of the parasites will regain motility within 24 hours after exposure. The results were compared with a similar test conducted on a strain with no previous history of treatment failures, neither by H₂O₂, nor by any other chemical (LS Alta). The median immobilizing concentrations of H₂O₂ in mg/l (with 90 % CI) for these two strains were:

| | |
|---|---|
| LS H₂O₂ | 1708 (1486-1962) |
| LS Alta | 209 (157-277) |

The mechanism behind the observed reduced sensitivity has not been determined previously. Studies at NMBU, School of Veterinary Science, have indicated overexpression of a metabolic enzyme catalase to be a factor. Based on these findings, catalase genes were sequenced using degenerated primers, and PatoGen has developed a TaqMan qPCR assay for the expression of this catalase gene. Several studies have been performed to confirm the correlation between H₂O₂-resistance and the overexpression of this catalase gene.

### Example 1: H₂O₂-resistance is related to catalase gene expression

In order to determine if resistance towards H₂O₂ in sea lice is related to the expression level of the catalase gene, the genome sequence for the Lepeophtheirus salmonis catalase gene were sequenced using degenerated primers based on the known sequence for the catalase gene in *Caligus clemensi.* (http://www.ncbi.nlm.nih.gov/nucleotide/225719453?report=genbank&log$=nuclalign&bla st_rank=1&RID=9VZC439Y01R). In order to quantify the expression level of the catalase gene in sea lice strains with known resistance status toward H₂O₂-treatments, a PCR-assay based upon the binding of the fluorescent dye SYBR-Green I into the PCR product (PE Applied Biosystems, Warrington, UK) was used. The primers used in this study are listed in table 1.

**Table 1: Primers used in SYBR-Green I assay for study of expression level of the catalase gene in sea lice (Lepeophtheirus salmonis).**

| Name | Primer sequence | length | GC% | Tm | product length |
|---|---|---|---|---|---|
| CATALASE_F6 | CCACAGAACAACTTGCCAAC SEQ ID No. 2 | 20 | 50 | 59.1 | 157 |
| CATALASE_R6 | GCCATTTCGTCCATAAATGC SEQ ID NO. 3 | 20 | 45 | 60.3 | |

### Results

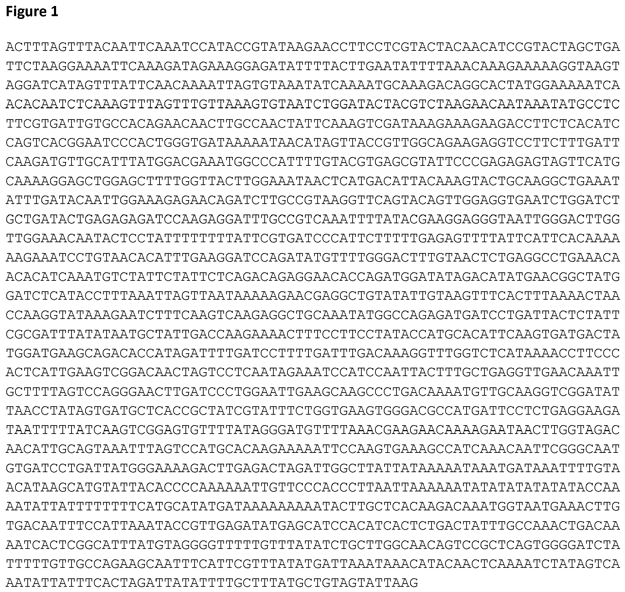

The catalase gene sequence from the *Lepeophtheirus salmonis* is presented in figure 1.

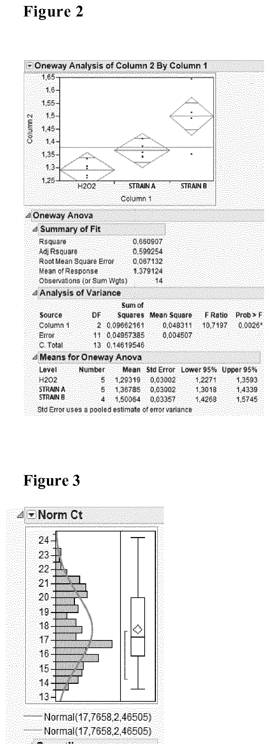

The difference in catalase expression is significant between the H₂O₂-resistant strain (labelled H₂O₂), and the other two strains when using the calculated ratio value for catalase / elongation factor in each sample (Figure 2). Thus, there is strong indication that the expression level of the catalase gene is suitable for differentiating between lice resistant and sensitive to H₂O₂ treatment.

**Table 2: Expression level of the catalase gene in sea lice strains with known resistance status toward H₂O₂-treatments, using a PCR-assay based upon the binding of the fluorescent dye SYBR-Green I**

| | | | FIRST PARALEL | | SECOND PARALEL | | THIRD PARALEL | | Ratio |
|---|---|---|---|---|---|---|---|---|---|
| Strain | **Nr.** | **RNA quality (ng/ul)** | **Catalase** | **ELF1** | **Catalase** | **ELF1** | **Catalase** | **ELF1** | **Kat/ELF** |
| H₂O₂ | 6 | 247 | 26,51 | 20,89 | 26,31 | 20,73 | 39,665 | 31,255 | 1,269 |
| H₂O₂ | 7 | 87 | 27,96 | 21,49 | 27,85 | 21,23 | 41,885 | 32,105 | 1,305 |
| H₂O₂ | 8 | 158 | 25,63 | 19,76 | 25,34 | 19,54 | 38,3 | 29,53 | 1,297 |
| H₂O₂ | 9 | 168 | 27,43 | 20,46 | 27,28 | 20,63 | 41,07 | 30,775 | 1,335 |
| H₂O₂ | 10 | 184 | 25,29 | 20,3 | 26,06 | 20,19 | 38,32 | 30,395 | 1,261 |
| | | | | | | | | | |
| Strain A | 21 | 173 | 27,69 | 19,4 | 27,16 | 19,62 | 41,27 | 29,21 | 1,413 |
| Strain A | 22 | 262 | 27,52 | 20,31 | 26,98 | 19,72 | 41,01 | 30,17 | 1,359 |
| Strain A | 23 | 154 | 28,2 | 20,86 | 27,28 | 20,62 | 41,84 | 31,17 | 1,342 |
| Strain A | 25 | 133 | 27,1 | 19,56 | 26,9 | 19,48 | 40,55 | 29,3 | 1,384 |
| Strain A | 26 | 338 | 26,75 | 20,1 | 26,13 | 19,19 | 39,815 | 29,695 | 1,341 |
| | | | | | | | | | |
| Strain B | 21 | 192 | 31,8 | 20,12 | 35,6 | 20,13 | 49,6 | 30,185 | 1,643 |
| Strain B | 22 | 161 | 31,3 | 21,02 | 31,09 | 20,72 | 46,845 | 31,38 | 1,493 |
| Strain B | 23 | 67 | 34,11 | 25,41 | 34,19 | 24,87 | 51,205 | 37,845 | 1,353 |
| Strain B | 24 | 125 | 33,11 | 22,02 | 32,99 | 21,51 | 49,605 | 32,775 | 1,514 |

### Example 2: Lice mortality after experimental exposure correlate with the expression of a catalase gene, showing that H₂O₂ resistance is related to the expression of the catalase gene in sea lice

The aim of this study was to test if the expression level of a catalase gene using PatoGen's TaqMan qPCR-assay correlate with the ability of sea lice (*L. salmonis*) to withstand H₂O₂-exposure. PCR-analyses were performed on dead and surviving parasites exposed to hydrogen peroxide in two experiments;
1. Sea lice infecting salmon were treated with 1500 mg hydrogen peroxide for 20 minutes.
2. Parasites in tanks without fish were treated with 2300 mg/l hydrogen peroxide for 20 minutes.

Based on the knowledge that the expression of the catalase gene is an important determinant for resistance towards H₂O₂-treatment in sea lice, PatoGen Analyse AS developed a sensitive Real-Time PCR (TaqMan) 5'-nuclease assay to quantify the expression level of the catalase gene. Real-time PCR has become a well-established procedure for quantifying levels of gene expression (c.f. e.g. de Kok JB, van Balken MR, Roelofs Rwhm, van Aarssen Yawg, Swinkels DW, Gunnewiek Jmtk: Quantification of hTERT mRNA and telomerase activity in bladder washings of patients with recurrent urothelial cell carcinomas. Clin. Chem. 2000, 46:2003-2007., Homey B, Dieu-Nosjean MC, Wiesenborn A, Massacrier C, Pin JJ, Oldham E, Catron D, Buchanan ME, Muller A, Malefyt RD, Deng G, Orozco R, Ruzicka T, Lehmann P, Lebecque S, Caux C, Zlotnik A: Up-regulation of macrophage inflammatory protein-3 alpha/CCL20 and CC chemokine receptor 6 in psoriasis. J. Immunol. 2000, 164:6621-6632., Kubo A, Nishitani V, Minamino N, Kikumoto K, Kurioka H, Nishino T, Iwano M, Shiiki H, Kangawa K, Dohi K: Adrenomedullin gene transcription is decreased in peripheral blood mononuclear cells of patients with IgA nephropathy. Nephron 2000, 85:201-206.).The catalase assay were used together with an assay towards the Elongation factor 1 C (E1α), and results for the E1α-assay were used for normalization in order to standardize the results from different runs of the catalase-PCR-assay. The catalase-PCR-assay, in combination with the E1α-assay, was successfully applied to differentiate between H₂O₂-resistant and sensitive sea lice. The analyses were run in a commercially available thermocycling fluorimeter (Applied Biosystems 7500 Real-Time PCR System) at PatoGen Analyse AS laboratory in Ålesund. The assays were used qualitatively to determine the relative amount of the gene in individual samples, and samples from different life stages of sea lice were used. The Real-Time PCR-assay in combination with the E1α is ideal for the differentiating between H₂O₂-resistant and sensitive sea lice in the aquaculture industry.

### TREATMENT TRIALS

Two treatment trials were performed where the present inventors analyzed lice surviving H₂O₂-treatment in the laboratory, and compared catalase gene expression levels in the population before treatment with the catalase gene expression level in surviving lice. In the first trial, H₂O₂-treatment were performed with sea lice attached to fish, while the second H₂O₂-reatment trial were performed using sea lice allowed to settle in a tank without fish. In both trials, lice affected by the treatment were collected in a system specially designed for these trials, for collecting lice in the outlet water from the tank.

Several strains were used in the study, which was conducted in the Licelab at the Sea lice Research Centre in Bergen. These included fully sensitive strains, a strain resistant to pyrethroids, a strain resistant to pyrethroids, organophosphates and emamectin benzoate, and a strain resistant to pyrethroids, organophosphates, emamectin benzoate and hydrogen peroxide. The strains were hatched at the same time, and the fish infested with a mixture of copepodites from all strains. The fish were divided into two groups kept in separate tanks.

### Pre-test treatment

After development of pre-adult parasites, the fish were treated with cypermethrin, 15 µg/l for 30 minutes (Betamax) in another experiment. Two tanks were used for the original experiment. Remaining fish from this first experiment with surviving sea lice were used in the hydrogen peroxide treatment experiment. Though, sea lice included in this experiment had been pre-treated with cypermethrin.

### Treatment trial 1: Fish experiment ("FISK")

Fish with a mixture of sensitive and resistant sea lice as described above, were treated with 1500 mg hydrogen peroxide for 20 minutes. Parasites that fell off the fish were collected in a filter on the outlet from the tank (immob). Parasites that were still attached to the fish were sampled from anesthetized fish 24 hours after the exposure (lev). In theory, the most resistant lice should be unaffected by the H₂O₂-treatment, and will remain attached to the fish, while the sensitive sea lice should be affected by treatment, loosen its grip of the fish, and is collected in the outlet water. We performed PCR-analyses for the expression level of the catalase gene on all lice from this trial.

### Treatment trial 2: Parasite experiment ("PARASITT")

A mixed population of sea lice from sensitive and resistant populations as described above was allowed to settle in a tank without fish. The sea lice settled on the plastic surface of the tank walls. The lice in the tank were exposed to 2300 mg/l H₂O₂ for 20 minutes, and lice that lost its grip to the tank walls were collected in the water outlet of the tank with a filter unit. Surviving parasites attached to the wall of the incubator were sampled (lev). Immobilized parasites on the bottom of the incubator were also sampled (immob). Resistant lice would be unaffected by the exposure to H₂O₂, and still be attached to the tank walls, while the sensitive lice would lose its grip to the tank walls, and come out of the tank with the outlet water. We performed PCR-analyses for the expression level of the catalase gene on all lice from this trial.

### Nucleic acid purification

In PatoGens laboratory, RNA and/or DNA were extracted from samples by methods well known to the skilled person. In short, tissue samples were transferred to Micro Collection Tubes and lysed and homogenized using QIAzol Lysis Reagent, steel beads and vigorous shaking using a TissueLyser system, followed by nucleic acid extraction using an RNAeasy kit (Qiagen) or DNAeasy kit (Qiagen), all according to the manufacturer's instructions, and by methods well known to the skilled person. Chloroform were added to the samples and shaken vigorously. After resting and centrifuging, the relevant liquid phase were collected for further extraction of either RNA or DNA by vacuum technology using a Qiagen robot system, all according to the manufacturer's instructions, and by methods well known to the skilled person. Finally, nucleic acids were eluted in 25 ml of elution buffer and used for PCR by methods well known to the skilled person.

### Real-Time PCR

Based on the newly sequenced *Lepeophtheirus salmonis* catalase gene, TaqMan® MGB Probe SNP Genotyping Assays using TaqMan® 5' nuclease assay chemistry for detecting and quantifying the catalase gene in purified genomic DNA or RNA were established. Primers and probes are listed in table 3. The primers and probes used were ordered from Life Technologies Corporation. One-step amplification (45 cycles) was performed on an Applied Biosystems 7500 Real-Time PCR System performed at PatoGen Analyse AS laboratory in Ålesund, all according to the manufacturer's instructions, and by methods well known to the skilled person. The assays were used qualitatively to determine the expression level of the catalase gene in individual samples.

**Table 3: Primers and probes used to quantify the expression of the catalase gene in sea lice.**

| **Name** | **Forward primer** | **Revers primer** | **Probe** |
|---|---|---|---|
| **Cat1** | Kata-ST-F | Kata-ST-R | Kata-ST-P |
| Lepeopht heirus salmonis | TCACATCCAGTCACGGAATCC (SEQ ID No.4) | ACCTCTTCTGCCAACGGTAACTA (SEQ ID No. 5) | ACTGGGTGATAAAAAT (SEQ ID No. 6) |
| **Cat2** | Kata-St1-F | Kata-ST1-R | Kata-ST1-P |
| Lepeophth eirus salmonis | GCCTGAAACAACACATCAAATGTC (SEQ ID No. 7) | GCCGTTCATATGTCTATATCCATCTG (SEQ ID No. 8) | TCTATTCTCAGACAGAGGAA (SEQ ID No. 9) |
| **Cat3** | Kata-ST2-F | Kata-ST2-R | Kata-ST2-P |
| Lepeophth eirus salmonis | ACCGTTGAGATATGAGCATCCA (SEQ ID No. 10) | TGCCGAGTGATTTTGTCAGTTT (SEQ ID No. 11) | ATCACTCTGACTATTTGC (SEQ ID No. 12) |

### RESULTS

The result from Real-Time PCR-assay is interpreted by using the Ct-value for the catalase assay, normalizing the values toward the Ct-value for the E1α (Frost & Nilsen 2003, Validation of reference genes for transcription profiling in the salmon louse, Lepeophtheirus salmonis, by quantitative real-time PCR, Veterinary Parasitology 118 (2003) 169-174 Short) for the same individual lice by dividing the catalase Ct-value with the Ct-value for the E1α for the individual lice, and multiplying with the average Ct-value for the E1α for about 800 lice previously analyzed. The normalized Ct-value is used to establish the resistance level of individual lice and for lice populations. Sensitive sea lice have high Ct-values, indication a low expression of the catalase gene. Genetically resistant sea lice have lower Ct-values, indicating an elevated expression of the catalase gene, and a higher capacity to tolerate H₂O₂, and thus a higher resistance towards H₂O₂.

### Statistical analyses

All normalized Ct values were first examined for normality using JMP 10.0.0. (SAS Institute). The distribution was clearly bi-phasic (Fig. 3) and could not be transformed to normality by log- or root transformation. Thus, the non-parametric Wilcoxon test of rank sums was used to examine the difference between the groups.

### RESULTS

The distribution of normalized Ct-values for catalase in parasites from the groups "Fisk" (treatment of fish with lice) and "Parasitt" (treatment of lice only) are presented in Figure 3. The median value was almost identical for both groups (norm. Ct: 17.2 and 17.3, respectively).

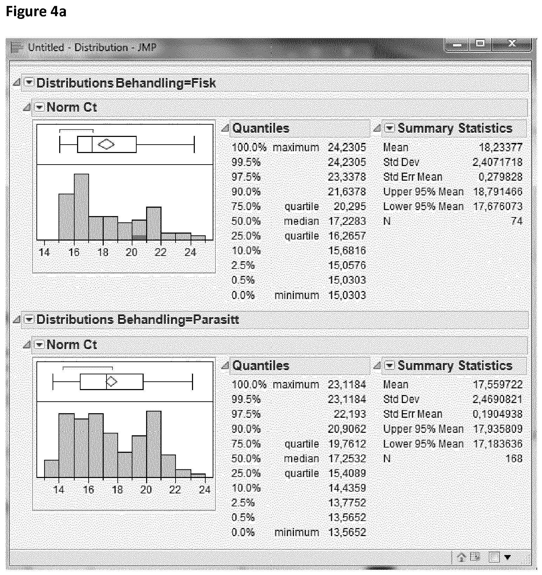

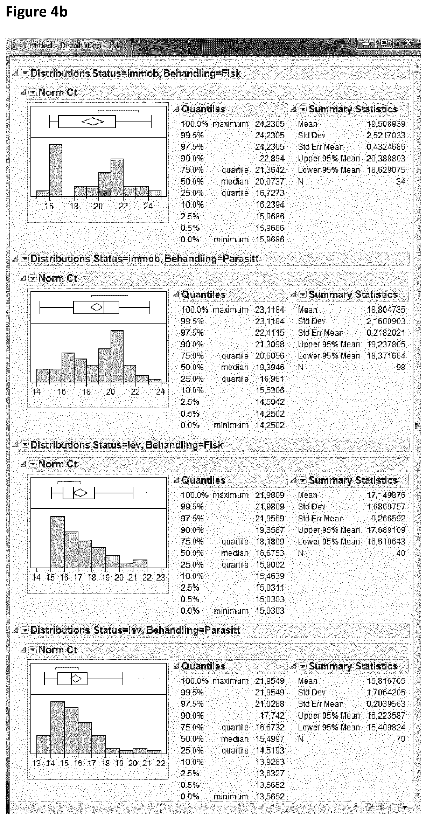

Thereafter, the distribution of normalized Ct-values for catalase between alive (lev) and immobilized (immob) parasites from the two groups "Fisk" (treatment of fish with lice) and "Parasitt" (treatment of lice only) was examined (Figure 4). The results demonstrated similar normalized Ct-values for both live parasites (median: 16.7 and 15.5 for the "Fisk" and the "Parasitt" groups, respectively) and immobilized parasites (median: 20.1 and 19.4 for the "Fisk" and the "Parasitt" groups, respectively).

Then, the Wilcoxon test of rank sums was applied to test whether or not there was a statistically significant difference between surviving (lev) and immobilized (immob) parasites within each treatment group (parasites on the fish, "Fisk" or parasites alone, "Parasitt") (Figure 4). The analysis demonstrated a highly significant difference between normalized Ct-values for both treatment groups (p<0.0001 for both treatments).

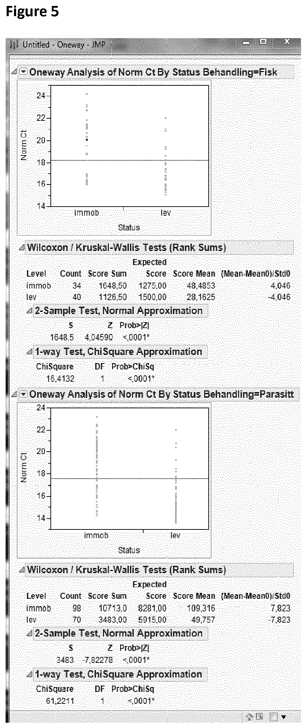

Finally, a Wilcoxon test of rank sums was performed to see if there was a statistically significant difference in normalized Ct-values in surviving (lev) parasites between the treaments groups (parasites on fish, "Fisk" or parasites alone, "Parasitt"). Thereafter, the test was repeated for immobilized (immob) parasites (Figure 5). For the immobilized parasites, no significant difference was evident (p=0.1495). For the alive parasites, there was a highly significant difference (p<0.0001) with highest Ct-values (lowest expression) in the parasites on fish.

Finally we used logistic regression to test the effect of normalized Ct-values on the probability of lice being immobilized in the two experiments, according to the general linear model: logit (y) = b0 + b1x,
where b0 is the intercept and b1 is the coefficient of the effect of normalized Ct-values (x). Statistics for these analyses from the two experiments is given in Table 4.

**Table 4. Summary statistics for the logistic regression analyses of the experiments on H₂O₂ treatment of fish ("Fisk") and parasites alone ("Parasitt").**

| Experiment | Coefficients | Estimate | Std. Error | z value | P |
|---|---|---|---|---|---|
| "Parasitt" | Intercept | -11.73 | 1.829 | -6.413 | < 0.001 |
| | Norm Ct-value | 0.703 | 0.108 | 6.499 | < 0.001 |
| | | | | | |
| "Fisk" | Intercept | -9.15 | 2.340 | -3.908 | < 0.001 |
| | Norm Ct-value | 0.493 | 0.128 | 3.837 | < 0.001 |

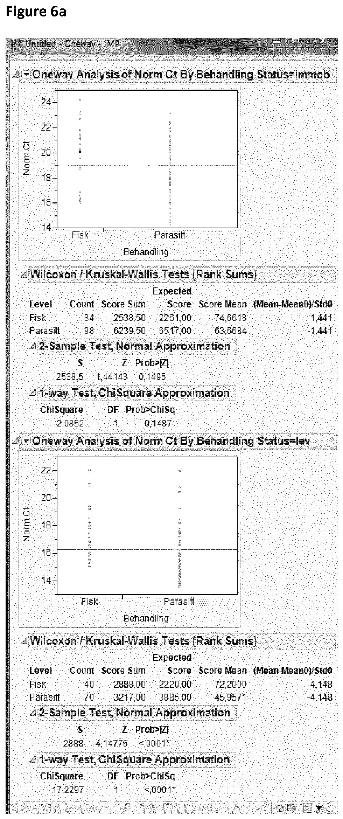

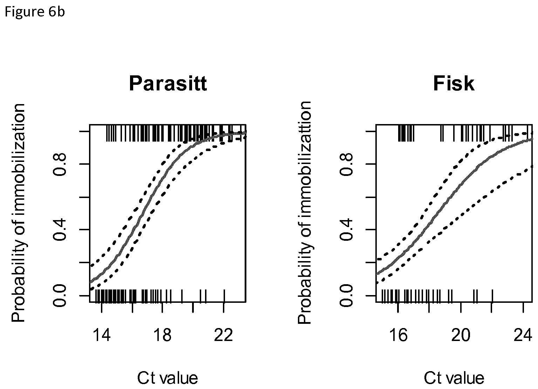

The results from the logistic regression analyses are presented in Figure 6b.

### DISCUSSION

The analyses demonstrated highly significant differences between normalized Ct-values in surviving and immobilized parasites, both after H₂O₂ exposure of parasites on fish and H₂O₂ exposure of parasites alone. The highest Ct-values (lowest expression of the gene) were found in immobilized parasites in all cases. This observation demonstrates that a salmon louse with a higher expression of the enzyme catalase has a higher likelihood of surviving a treatment with hydrogen peroxide than parasites with a lower expression of the catalase enzyme.

In the study, significantly lower Ct-values (higher expression of the catalase gene) was found in live parasites exposed to hydrogen peroxide alone compared to live parasites exposed to hydrogen peroxide while still attached to the fish. Thus, an elevated expression of the catalase gene is related to resistance toward H₂O₂-treatment, and lice affected by H₂O₂-treatment have a lower expression of the catalase gene than resistant lice.

The difference in the mean normalized Ct-value was though only 1.2. The most likely reason is that the parasites exposed alone were exposed to a higher H₂O₂ concentration (2300 ppm) compared to the parasites exposed while still on the fish (1500 ppm). Thus, a higher proportion of parasites with intermediate sensitivities were immobilized in the group exposed alone compared to the group exposed on fish.

The Real-Time PCR -assay showed surprisingly good correlation with resistance status towards H₂O₂, and we believe that it will serve as a practical tool in the differentiation between H₂O₂ sensitive and resistant sea lice at individual and population level.

Thus, using the expression level of the catalase gene quantified by one of the Real-Time PCR-assays described here represent an ideal tool for differentiating between H₂O₂ resistant and sensitive sea lice in the aquaculture industry. Also, the prevalence of sensitive versus resistant sea lice in a population can be used to predict the best possible outcome of a treatment using H₂O₂ in the population. Also, the technique can become an important tool for optimizing sea lice treatments using H₂O₂, combination treatments using H₂O₂ in combination with other insecticides or other measures to reduce sea lice infection pressure, and to monitor the resistant status of populations of sea lice before treatment.

### Example 3: Enzymatic activity of the enzyme catalase and the expression of the gene coding for catalase in the salmon louse, correlate with resistance towards H2O2 in sea lice

The aim of this study was to test if the expression level of a catalase enzyme correlates with the expression of a catalase gene using PatoGens TaqMan qPCR-assay, and to establish if this expression correlates with the ability of sea lice (*L. salmonis*) to withstand H₂O₂-exposure. Enzyme quantification and PCR-analyses were performed on parasites collected in Norwegian aquaculture sites known to have varying ability to withstand exposure to hydrogen peroxide.

### MATERIALS AND METHODS

### SALMON LICE STRAINS

### Ls Alta

This strain originated from a salmon farm located at the island Seiland in the Alta fjord in Finnmark county. It was collected in 2010 and has been kept in continuous culture at the NIVA marine research station Solbergstrand since then. It has been cultured for about 10 generations. There has been no history of insensitivity towards any chemicals at the farm or in the district from which these parasites originate.

### Ls H₂O₂

This strain originated from a salmon farm in Vikna, Nord-Trøndelag. It was collected in 2013 and had been cultured for only one generation when the study was performed, on the offsprings of the original strain. At the site and in the district, there had been increasing problems with insensitivity of salmon lice towards available chemicals. The latest problem was with hydrogen peroxide, where the efficacy of several treatments was substantially lower than expected.

The parasites included were adult females. After sampling, each parasite was split along the mid-line with a scalpel. One half was immediately frozen at -80 degrees in a 0.5 ml Eppendorf vial and later used for the enzymatic assay. The other half was put on RNAlater, shipped to PatoGen Analyse AS, and used for the TaqMan PCR assay.

### ASSAYS

### Catalase activity assay

To determine if the catalase enzyme activity is important for resistance towards H₂O₂ in sea lice, an enzymatic assay using a standardized commercial kit for catalase activity was established. The catalase activity was measured in each split parasite. A commercial kit (Oxyselect catalase activity kit) was used according to the manufacturer's instructions in a 96 well microplate. The activity was read on an Epoch spectrophotometer, BioTek, USA, Gen5 verion 2.00 software. The activity was normalized against the protein content in the homogenate. Protein content was measured on a Take3 plate (BioTek) with a build-in protein calculation calibrated against bovine serum albumin on the same microtitre plate reader.

### TaqMan assay

The expression of the catalase gene was measured using a TaqMan assay developed by PatoGen Analyse AS following procedures as described in example 2. The results were normalized against the expression of the elongation factor in the samples, also as described in example 2.

### RESULTS

The correlation between normalized Ct-values and catalase activities (corrected for protein) were analysed using the statistical module incorporated in Excel (Microsoft). The correlation of all catalase activity values (U/µg protein) versus all Ct-values demonstrated a Pearson correlation factor of -0.3524. A regression analysis (Excel) demonstrated the association between enzyme activity and CT-values to be significant (p=0.016, Fig. 1).

**Table 4: Output from the regression analyses of catalase activity versus gene expression (normalized Ct-values). Multiple R = Pearson's correlation coefficient.**

| SUMMARY OUTPUT | | | | | | | | |
|---|---|---|---|---|---|---|---|---|
| | | | | | | | | |
| *Regression Statistics* | | | | | | | | |
| Multiple R | 0,35240783 | | | | | | | |
| R Square | 0,12419128 | | | | | | | |
| Adjusted RS | 0,10428654 | | | | | | | |
| Standard Err | 1,56697035 | | | | | | | |
| Observation | 46 | | | | | | | |
| | | | | | | | | |

| ANOVA | | | | | | | | |
|---|---|---|---|---|---|---|---|---|
| | *df* | *SS* | *MS* | *F* | *Significance F* | | | |
| Regression | 1 | 15,319905 | 15,319905 | 6,23928055 | 0,01630628 | | | |
| Residual | 44 | 108,037427 | 2,45539608 | | | | | |
| Total | 45 | 123,357332 | | | | | | |
| | | | | | | | | |

| | *Coefficients* | *tandard Erro* | *t Stat* | *P-value* | *Lower 95%* | *Upper 95%* | *Lower 95,0%* | *Upper 95%* |
|---|---|---|---|---|---|---|---|---|
| Intercept | 20,3217353 | 0,41708976 | 48,7226901 | 6,3368E-40 | 19,4811461 | 21,1623244 | 19,4811461 | 21,1623 |
| X Variable 1 | -0,36200971 | 0,14492822 | -2,49785519 | 0,01630628 | -0,65409335 | -0,06992607 | -0,65409335 | -0,06992 |

The data were grouped by strain, and the differences in mean catalase activity (U/µg protein) and mean normalized Ct-values were calculated using the statistical software JMP (SAS Institute). An analysis of variance was used for testing each parameter against strain.

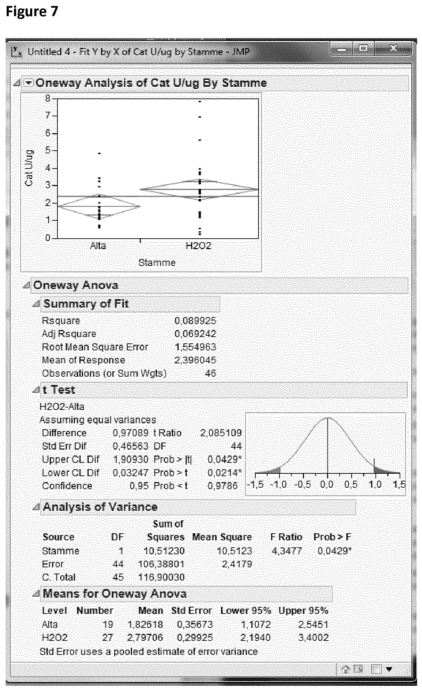

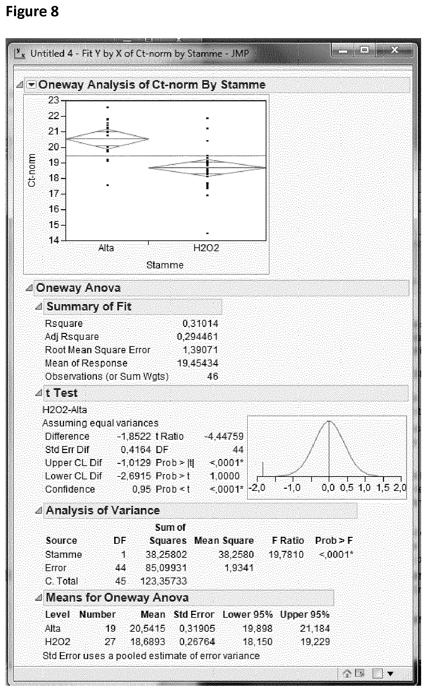

For catalase activity, a higher enzyme activity was demonstrated in the H₂O₂ strain compared to the Alta strain. The difference was statistically significant (p= 0.0429, Fig. 7). For the normalized Ct-values, lower values were found in the H₂O₂ strain compared to the Alta strain. This difference was statistically highly significant (p<0.0001, Fig. 8).

### DISCUSSION

The observed difference in sensitivity between these two strains, as demonstrated by bioassays, was associated with the expression of the gene coding for the catalase enzyme and the activity of this enzyme. The correlation between activity and expression was not perfect, but still significant. The enzyme activity assay used showed a big variation between parallel samples, thus the estimated activities were somewhat inaccurate.

In conclusion, an association between bioassay results, enzymatic activity of the catalase enzyme and expression of the gene coding for catalase were demonstrated.

### Example 4: Expression levels of the gene coding for the enzyme catalase in field population of sea lice.

The aim of this study was to test if the expression of the catalase gen in sea lice populations correlates with the known geographic distribution of resistance towards H₂O₂, or to areas with high treatment frequencies using H₂O₂. Several strains of salmon lice were sampled from different sites along the Norwegian coast.

### MATERIALS AND METHODS

### SALMON LICE STRAINS

Salmon lice *(Lepeophtheirus salmonis),* different developmental stages (pre-adult 1, pre-adult 2 and adult lice of both sexes) were sampled from fish farms along the west coast of Norway with unknown resistance status towards H₂O₂. Sea lice were collected using forceps, and 60 lice per site were conserved in 70% ethanol and kept at 4°C. Samples were sent refrigerated to PatoGen by express mail carrier.

The strains were labelled by county and location within the county (N, M or S for north, middle and south, 1, 2, ... for different sites).

### TaqMan ASSAY

The expression of the catalase gene was measured using a TaqMan assay developed by PatoGen Analyse AS following procedures as described in example 2. The results were normalized against the expression of the elongation factor in the samples, also as described in example 2.

### STATISTICAL ANALYSES

All normalized Ct values from each site were first examined separately for normality using JMP 10.0.0. (SAS Institute). The distribution within a site could for most sites be described by a normal distribution according to the Shapiro-Wiik W test. The strains not displaying a normal distribution were Nordland N2, Nordland M2, S-Trøndelag M and Rogaland N. As 9 of the 13 sample sets followed normal distribution, ANOVA was used to examine differences in mean values between the strains.

Furthermore, recursive partitioning was used (JMP 10.0.0.) to split the strains into three clusters:
1) High expression of the catalase gene (low Ct values)
2) Intermediate expression of the catalase gene (intermediate Ct values)
3) Low expression of the catalase gene (high Ct values)

### RESULTS

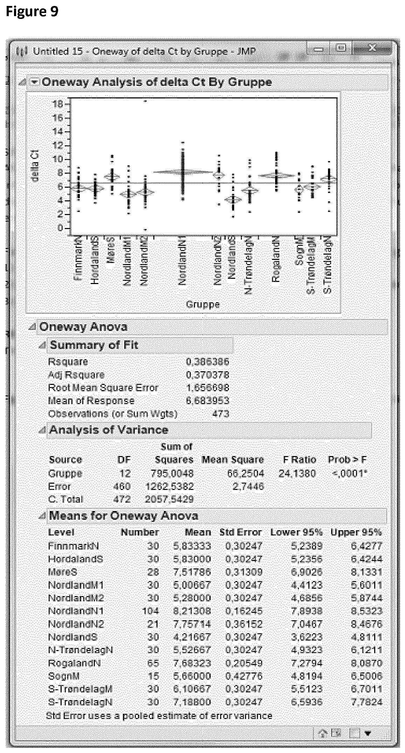

The distribution within each group and the ANOVA analysis of normalized CT values versus strain is presented in Figure 9, and demonstrates that the confidence intervals for several strains are not overlapping, indicating a significant difference in the expression.

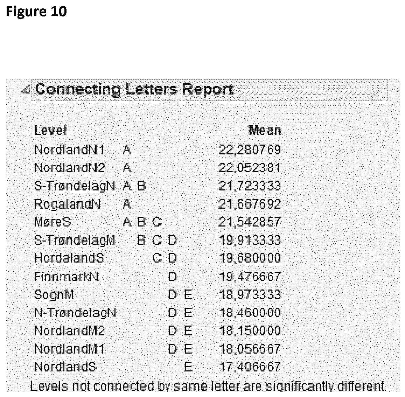

Thereafter, the expression values for all strains were compared using the Tukey-Kramer HSD test. The results are presented in Figure 10, and demonstrate in which strains the expression levels differed significantly (strains not sharing the same letter).

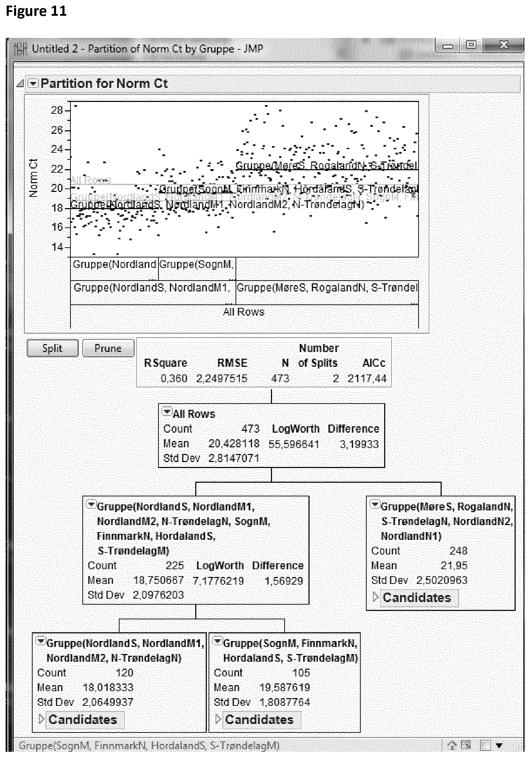

Finally, the clustering of results was examined using recursive partitioning. The results are presented in Figure 11, and demonstrated that the strains could be separated into

| | |
|---|---|
| 1) High expression: | N-Trøndelag N, Nordland S, Nordland M1, Nordland M2, |
| 2) Intermediate expression: | Hordaland S, Sogn M, S-Trøndelag M, Finnmark N |
| 3) Low expression: | Rogaland N, S-Trøndelag N, Nordland N1, Nordland N2 |

### DISCUSSION

The analyses demonstrated that there were significant differences between salmon lice strains in their expression of the catalase gene. The highest expressions were found in strains originating from the county N-Trøndelag and the south and middle part of the county Nordland. This is as expected, as these are areas where reduced treatment efficacy by hydrogen peroxide has been reported. The lowest expression of the catalase enzyme was found in samples from the northern part of the county Nordland and some scattered sites further south, all areas where there has been little or no use of hydrogen peroxide as a delousing agent. However, somewhat surprising, the strain from Finnmark, where hydrogen peroxide has never been used to treat for salmon lice, clustered in the intermediate group.

In conclusion, there seems to be a strain-specific variation in the expression of the enzyme which is fairly well correlated to the use of hydrogen peroxide as a delousing agent in Norway.

## Claims

1. Method for the detection of hydrogen peroxide resistance in one or more sea lice selected from the group consisting of *Lepeophtheirus salmonis, Caligus clemensei, Caligus elongatus,* and *Caligus rogercresseyi* comprising the steps of:
a) collecting one or more sea lice from infested fish or water samples;
b) isolating genomic material from any life stage of the collected sea lice; and
c) determining the expression level of a catalase gene of collected sea lice.

2. Method according to claim 1, wherein said catalase gene has a sequence as depicted in SEQ ID No. 1, SEQ ID No. 13 or SEQ ID No. 14, or variants or fragments thereof being at least 70 % identical with SEQ ID No. 1 SEQ ID No. 13 and SEQ ID No. 14, respectively.

3. Method according to claim 1, wherein said catalase gene has a sequence as depicted in SEQ ID No. 1, SEQ ID No. 13 or SEQ ID No. 14, or variants or fragments thereof being at least 80 % identical with SEQ ID No. 1 SEQ ID No. 13 and SEQ ID No. 14, respectively.

4. Method according to any of the above claims, comprising the steps of detecting increased RNA-expression levels associated with hydrogen peroxide (H₂O₂) resistance in the sea lice to be analyzed, wherein said sea lice are resistant to hydrogen peroxide (H₂O₂) resistance if having elevated levels of catalase RNA-expression.

5. Method according to any of the above claims, comprising in step c:
- providing one or more isolated oligonucleotide sequence(s) comprising at least 8 contiguous nucleotides of the sequence SEQ ID No. 1, SEQ ID No. 13 or SEQ ID No. 14 or a complementary oligonucleotide of SEQ ID No. 1, SEQ ID No. 13 or SEQ ID No. 14, respectively; and
- determining the expression level of the catalase gene as depicted in SEQ ID No. 1, SEQ ID No. 13 or SEQ ID No. 14, or variants or fragments thereof being at least 70 % identical with the entire length of SEQ ID No. 1 SEQ ID No. 13 and SEQ ID No. 14, respectively, in the collected sea lice by measuring the catalase RNA expression level in the collected sea lice, wherein said sea lice is resistant to hydrogen peroxide (H₂O₂) if having elevated levels of catalase RNA-expression compared with a non-resistant sea lice.

6. Method according to claim 5, wherein said determination is performed using a primer selected from the group consisting of SEQ ID No.'s 2, 3, 4, 5, 7, 8, 10 and 11, or by using at least one probe selected from the group consisting of SEQ ID No.'s 6, 9 and 12, or by using a primer selected from the group consisting of SEQ ID No.'s 15, 16, 18 and 19, or by using at least one probe selected from the group consisting of SEQ ID No.'s 17 and 20.

7. Method for the detection of hydrogen peroxide resistance in one or more sea lice selected from the group consisting of *Lepeophtheirus salmonis, Caligus clemensei, Caligus elongatus,* and *Caligus rogercresseyi* comprising the steps of:
a) collecting sea lice from infested fish or water samples;
b) homogenization of sea lice tissue samples from any life stage of collected sea lice;
c) determining the catalase activity, wherein said one or more sea lice is resistant to hydrogen peroxide (H₂O₂) if the catalase activity is elevated.

8. Method according to claim 7, wherein the determining of catalase activity step c) is performed by contacting a sample isolated from the crustaceans to be analyzed with a pre-determined amount of hydrogen peroxide, and then with horseradish peroxidase and a chromophore, resulting in the formation of a chroma signal, and wherein the decomposition of hydrogen peroxide is proportional with the level of catalase in the sample.

9. Method for characterizing the hydrogen peroxidase sensitivity of one or more sea lice, comprising the steps of
a) determining the expression level of the catalase gene according to SEQ ID No. 1 or a variant or fragment thereof in a biological sample comprising DNA or RNA of one or more sea lice, wherein said variant or fragment of SEQ ID No. 1 is the catalase gene of claim 1;
b) comparing the determined catalase expression level to (i) a control standard or (ii) the expression of the catalase gene or a variant or fragment thereof in a control sample;
c) determining the difference in expression level in the sample as compared to the control standard or control sample; and
d) characterizing the hydrogen peroxidase sensitivity of the one or more sea lice, wherein the one or more sea lice to be tested is resistant if having a higher expression level compared with the control standard or the control sample.

10. Method according to claim 9, comprising the steps of
- collecting sea lice from infested fish or water samples;
- isolating DNA or RNA from the collected sea lice;
- providing a pair of PCR primers specific for the catalase gene of SEQ ID No. 1, SEQ ID No. 13 or SEQ ID No. 14, respectively;
- performing PCR on the isolated DNA or RNA using the pair of PCR primers;
- determining the expression level of the catalase gene by comparing the level of catalase expression with the level of control standard or a catalase gene expression sample control.

11. An oligonucleotide probe comprising a nucleotide sequence, wherein the sequence consists of that selected from the group of SEQ ID No.'s 6, 9, and 12 and fragments and variants thereof both having at least 70 % sequence identity over the entire length of SEQ ID No.'s 6, 9, and 12.

12. An oligonucleotide probe comprising a nucleotide sequence according to claim 11, wherein the sequence comprises about 13 to 25 consecutive nucleotides.

13. An oligonucleotide primer comprising a nucleotide sequence, wherein the sequence comprises about 18 - 25 consecutive nucleotides and consist of that selected from the group of SEQ ID No.'s 2, 3, 4, 5, 7, 8, 10, and 11 and fragments and variants thereof both having at least 70 % sequence identity over the entire length of SEQ ID No.'s 2, 3, 4, 5, 7, 8, 10, and 11.

14. An oligonucleotide primer pair, wherein the sequences of said primer pair is selected from the groups consisting of SEQ ID No. 2 and 3, 4 and 5, 7 and 8, and, 10 and 11, and fragments and variants thereof both having at least 70 % sequence identity over the entire length of SEQ ID No. 2 and 3, 4 and 5, 7 and 8, and, 10 and 11.

15. Kit for detection of hydrogen peroxide (H₂O₂) resistance in crustaceans comprising at least one oligonucleotide probe, primer and/or primer pair according to claims 11-14.

16. Use in vitro of one or more isolated oligonucleotide sequence(s) comprising at least 8 contiguous nucleotides of the sequence SEQ ID No. 1, SEQ ID No. 13 or SEQ ID No. 14 or a complementary oligonucleotide of SEQ ID No. 1, SEQ ID No. 13 or SEQ ID No. 14, respectively, for the determination of hydrogen peroxide resistance in sea lice selected from the group consisting of *Lepeophtheirus salmonis, Caligus clemensei, Caligus elongatus,* and *Caligus rogercresseyi.*

## Patentansprüche

1. Verfahren zum Nachweis von Wasserstoffperoxid-Resistenz in einer oder mehreren Seeläusen, ausgewählt aus der Gruppe bestehend aus *Lepeophtheirus salmonis, Caligus clemensei*, *Caligus elongatus* und *Caligus rogercresseyi,* umfassend die folgenden Schritte:
a) Sammeln einer oder mehrerer Seeläuse von befallenen Fischen oder aus Wasserproben;
b) Isolieren von Genommaterial aus einem beliebigen Lebensstadium der gesammelten Seeläuse; und
c) Bestimmen des Expressionsniveaus eines Katalase-Gens der gesammelten Seeläuse.

2. Verfahren nach Anspruch 1, wobei das Katalase-Gen eine Sequenz, wie sie in SEQ ID Nr. 1, SEQ ID Nr. 13 oder SEQ ID Nr. 14 dargestellt ist, oder Varianten oder Fragmente davon, die zu mindestens 70% identisch mit SEQ ID Nr. 1, SEQ ID Nr. 13 bzw. SEQ ID Nr. 14 sind, aufweist.

3. Verfahren nach Anspruch 1, wobei das Katalase-Gen eine Sequenz, wie sie in SEQ ID Nr. 1, SEQ ID Nr. 13 oder SEQ ID Nr. 14 dargestellt ist, oder Varianten oder Fragmente davon, die zu mindestens 80% identisch mit SEQ ID Nr. 1, SEQ ID Nr. 13 bzw. SEQ ID Nr. 14 sind, aufweist.

4. Verfahren nach einem der vorstehenden Ansprüche, umfassend die Schritte des Nachweisens erhöhter RNA-Expressionsniveaus, die mit Wasserstoffperoxid(H₂O₂)-Resistenz in den zu analysierenden Seeläusen verbunden sind, wobei die Seeläuse resistent gegen Wasserstoffperoxid(H₂O₂)-Resistenz sind, wenn sie erhöhte Niveaus von Katalase-RNA-Expression aufweisen.

5. Verfahren nach einem der vorstehenden Ansprüche, umfassend in Schritt c:
- Bereitstellen einer isolierten oder mehrerer isolierter Oligonucleotid-sequenz(en), die mindestens 8 aufeinanderfolgende Nucleotide der Sequenz SEQ ID Nr. 1, SEQ ID Nr. 13 oder SEQ ID Nr. 14 oder eines jeweiligen komplementären Oligonucleotids von SEQ ID Nr. 1, SEQ ID Nr. 13 oder SEQ ID Nr. 14 umfassen; und
- Bestimmen des Expressionsniveaus des Katalase-Gens, wie es in SEQ ID Nr. 1, SEQ ID Nr. 13 oder SEQ ID Nr. 14 dargestellt ist, oder von Varianten oder Fragmente davon, die zu mindestens 70% identisch mit der Gesamtlänge von SEQ ID Nr. 1, SEQ ID Nr. 13 bzw. SEQ ID Nr. 14 sind, in den gesammelten Seeläusen, durch Messen des Katalase-RNA-Expressionsniveaus in den gesammelten Seeläusen, wobei die Seeläuse resistent gegen Wasserstoffperoxid (H₂O₂) sind, wenn sie verglichen mit einer nicht resistenten Seelaus erhöhte Niveaus von Katalase-RNA-Expression aufweisen.

6. Verfahren nach Anspruch 5, wobei die Bestimmung unter Verwendung eines Primers, ausgewählt aus der Gruppe bestehend aus den SEQ ID Nr. 2, 3, 4, 5, 7, 8, 10 und 11, oder unter Verwendung mindestens einer Sonde, ausgewählt aus der Gruppe bestehend aus den SEQ ID Nr. 6, 9 und 12, oder unter Verwendung eines Primers, ausgewählt aus der Gruppe bestehend aus den SEQ ID Nr. 15, 16, 18 und 19, oder unter Verwendung mindestens einer Sonde, ausgewählt aus der Gruppe bestehend aus den SEQ ID Nr. 17 und 20, durchgeführt wird.

7. Verfahren zum Nachweis von Wasserstoffperoxid-Resistenz in einer oder mehreren Seeläusen, ausgewählt aus der Gruppe bestehend aus *Lepeophtheirus salmonis, Caligus clemensei*, *Caligus elongatus* und *Caligus rogercresseyi,* umfassend die folgenden Schritte:
a) Sammeln von Seeläusen von befallenen Fischen oder aus Wasserproben;
b) Homogenisierung von Seelaus-Gewebeproben aus einem beliebigen Lebensstadium der gesammelten Seeläuse;
c) Bestimmen der Katalase-Aktivität, wobei die eine oder die mehreren Seeläuse gegen Wasserstoffperoxid (H₂O₂) resistent sind, wenn die Katalase-Aktivität erhöht ist.

8. Verfahren nach Anspruch 7, wobei das Bestimmen der Katalase-Aktivität in Schritt c) durchgeführt wird, indem eine aus den zu analysierenden Crustaceen isolierte Probe mit einer vorbestimmten Menge an Wasserstoffperoxid und anschließend mit Meerrettichperoxidase und einem Chromophor in Kontakt gebracht wird, was zur Bildung eines Farbsignals führt, und wobei die Zersetzung von Wasserstoffperoxid proportional zur Katalase-Konzentration in der Probe ist.

9. Verfahren zum Charakterisieren der Wasserstoffperoxidase-Sensitivität einer oder mehrerer Seeläuse, umfassend die folgenden Schritte:
a) Bestimmen des Expressionsniveaus des Katalase-Gens, das SEQ ID Nr. 1 oder einer Variante oder einem Fragment davon entspricht, in einer biologischen Probe, die DNA oder RNA einer oder mehrerer Seeläuse umfasst, wobei die Variante oder das Fragment von SEQ ID Nr. 1 das Katalase-Gen nach Anspruch 1 ist;
b) Vergleichen des bestimmten Katalase-Expressionsniveaus mit (i) einem Kontrollstandard oder (ii) der Expression des Katalase-Gens oder einer Variante oder eines Fragments davon in einer Kontrollprobe;
c) Bestimmen der Differenz des Expressionsniveaus in der Probe im Vergleich zum Kontrollstandard oder zur Kontrollprobe; und
d) Charakterisieren der Wasserstoffperoxidase-Sensitivität der einen oder mehreren Seeläuse, wobei die zu testende eine oder die zu testenden mehreren Seeläuse resistent sind, wenn sie ein im Vergleich zum Kontrollstandard oder zur Kontrollprobe höheres Expressionsniveau aufweisen.

10. Verfahren nach Anspruch 9, umfassend die folgenden Schritte:
- Sammeln von Seeläusen von befallenen Fischen oder aus Wasserproben;
- Isolieren von DNA oder RNA aus den gesammelten Seeläusen;
- Bereitstellen eines Paares von PCR-Primern, die jeweils spezifisch für das Katalase-Gen von SEQ ID Nr. 1, SEQ ID Nr. 13 oder SEQ ID Nr. 14 sind;
- Durchführen einer PCR an der isolierten DNA oder RNA unter Verwendung des Paares von PCR-Primern;
- Bestimmen des Expressionsniveaus des Katalase-Gens durch Vergleichen des Niveaus der Katalase-Expression mit dem Niveau des Kontrollstandards oder einer Kontrollprobe der Katalase-Genexpression.

11. Oligonucleotidsonde, umfassend eine Nucleotidsequenz, wobei die Sequenz aus einer solchen, die aus der Gruppe der SEQ ID Nr. 6, 9 und 12 ausgewählt ist, und aus Fragmenten und Varianten davon besteht, die beide zu mindestens 70% Sequenzidentität über die Gesamtlänge der SEQ ID Nr. 6, 9 und 12 aufweisen.

12. Oligonucleotidsonde, umfassend eine Nucleotidsequenz nach Anspruch 11, wobei die Sequenz etwa 13 bis 25 aufeinanderfolgende Nucleotide umfasst.

13. Oligonucleotidprimer, umfassend eine Nucleotidsequenz, wobei die Sequenz etwa 18 bis 25 aufeinanderfolgende Nucleotide umfasst und aus einer solchen, die aus der Gruppe der SEQ ID Nr. 2, 3, 4, 5, 7, 8, 10 und 11 ausgewählt ist, und aus Fragmenten und Varianten davon besteht, die beide zu mindestens 70% Sequenzidentität über die Gesamtlänge der SEQ ID Nr. 2, 3, 4, 5, 7, 8, 10 und 11 aufweisen.

14. Oligonucleotidprimerpaar, wobei die Sequenzen des Primerpaares ausgewählt sind aus den Gruppen bestehend aus SEQ ID Nr. 2 und 3, 4 und 5, 7 und 8 und 10 und 11 und aus Fragmenten und Varianten davon, die beide zu mindestens 70% Sequenzidentität über die Gesamtlänge von SEQ ID Nr. 2 und 3, 4 und 5, 7 und 8 und 10 und 11 aufweisen.

15. Kit zum Nachweis von Wasserstoffperoxid(H₂O₂)-Resistenz in Crustaceen, umfassend mindestens eine Oligonucleotidsonde, einen Primer und/oder ein Primerpaar nach den Ansprüchen 11-14.

16. Verwendung einer isolierten oder mehrerer isolierter Oligonucleotidse-quenz(en) in vitro, die mindestens 8 zusammenhängende Nucleotide der Sequenz SEQ ID Nr. 1, SEQ ID Nr. 13 oder SEQ ID Nr. 14 oder eines jeweiligen komplementären Oligonucleotids von SEQ ID Nr. 1, SEQ ID Nr. 13 oder SEQ ID Nr. 14 umfassen, zur Bestimmung der Wasserstoffperoxid-Resistenz in Seeläusen, ausgewählt aus der Gruppe bestehend aus *Lepeophtheirus salmonis, Caligus clemensei*, *Caligus elongatus* und *Caligus rogercresseyi.*

## Revendications

1. Procédé de détection d'une résistance au peroxyde d'hydrogène chez un ou plusieurs poux de mer sélectionnés dans le groupe consistant en *Lepeophtheirus salmonis, Caligus clemensei*, *Caligus elongatus*, et *Caligus rogercresseyi,* comprenant les étapes consistant à :
a) collecter un ou plusieurs poux de mer à partir d'un poisson infesté ou d'échantillons d'eau ;
b) isoler du matériel génomique à un quelconque stade de vie des poux de mer collectés ; et
c) déterminer le niveau d'expression d'un gène de catalase des poux de mer collectés.

2. Procédé selon la revendication 1, dans lequel ledit gène de catalase possède une séquence telle que représentée dans SEQ ID NO: 1, SEQ ID NO: 13 ou SEQ ID NO: 14, ou des variants ou fragments de celles-ci qui sont au moins 70 % identiques à SEQ ID NO: 1, SEQ ID NO: 13 et SEQ ID NO: 14, respectivement.

3. Procédé selon la revendication 1, dans lequel ledit gène de catalase possède une séquence telle que représentée dans SEQ ID NO: 1, SEQ ID NO: 13 ou SEQ ID NO: 14, ou des variants ou fragments de celles-ci qui sont au moins 80 % identiques à SEQ ID NO: 1, SEQ ID NO: 13 et SEQ ID NO: 14, respectivement.

4. Procédé selon l'une quelconque des revendications précédentes, comprenant les étapes consistant à détecter des niveaux d'expression d'ARN augmentés associés à une résistance au peroxyde d'hydrogène (H₂O₂) chez les poux de mer devant être analysés, où lesdits poux de mer sont résistants à une résistance au peroxyde d'hydrogène (H₂O₂) s'ils présentent des niveaux élevés d'expression d'ARN de catalase.

5. Procédé selon l'une quelconque des revendications précédentes, comprenant à l'étape c :
- la mise à disposition d'une ou de plusieurs séquence(s) oligonucléotidique(s) isolée(s) comprenant au moins 8 nucléotides contigus de la séquence SEQ ID NO: 1, SEQ ID NO: 13 ou SEQ ID NO: 14, ou d'un oligonucléotide complémentaire de SEQ ID NO: 1, SEQ ID NO: 13 ou SEQ ID NO: 14, respectivement ; et
- la détermination du niveau d'expression du gène de catalase tel que représenté dans SEQ ID NO: 1, SEQ ID NO: 13 ou SEQ ID NO: 14, ou de variants ou fragments de celui-ci qui sont au moins 70 % identiques sur toute la longueur de SEQ ID NO: 1, SEQ ID NO: 13 et SEQ ID NO: 14, respectivement, chez les poux de mer collectés en mesurant le niveau d'expression d'ARN de catalase chez les poux de mer collectés, où ledit pou de mer est résistant au peroxyde d'hydrogène (H₂O₂) s'il présente des niveaux élevés d'expression d'ARN de catalase par comparaison à un pou de mer non résistant.

6. Procédé selon la revendication 5, dans lequel ladite détermination est réalisée en utilisant une amorce sélectionnée dans le groupe consistant en SEQ ID NO: 2, 3, 4, 5, 7, 8, 10 et 11, ou en utilisant au moins une sonde sélectionnée dans le groupe consistant en SEQ ID NO: 6, 9 et 12, ou en utilisant une amorce sélectionnée dans le groupe consistant en SEQ ID NO: 15, 16, 18 et 19, ou en utilisant au moins une sonde sélectionnée dans le groupe consistant en SEQ ID NO: 17 et 20.

7. Procédé de détection d'une résistance au peroxyde d'hydrogène chez un ou plusieurs poux de mer sélectionnés dans le groupe consistant en *Lepeophtheirus salmonis, Caligus clemensei*, *Caligus elongatus*, et *Caligus rogercresseyi,* comprenant les étapes consistant à :
a) collecter des poux de mer à partir d'un poisson infesté ou d'échantillons d'eau ;
b) homogénéiser des échantillons tissulaires de poux de mer à un quelconque stade de vie des poux de mer collectés ;
c) déterminer l'activité de la catalase, où ledit un ou plusieurs poux de mer est résistant au peroxyde d'hydrogène (H₂O₂) si l'activité de la catalase est élevée.

8. Procédé selon la revendication 7, dans lequel la détermination de l'activité de la catalase à l'étape c) est réalisée par la mise en contact d'un échantillon isolé à partir des crustacés devant être analysés avec une quantité prédéterminée de peroxyde d'hydrogène, puis avec la peroxydase du raifort et un chromophore, en entraînant la formation d'un signal de chrominance, et dans lequel la décomposition du peroxyde d'hydrogène est proportionnelle au taux de catalase dans l'échantillon.

9. Procédé pour caractériser la sensibilité au peroxyde d'hydrogène d'un ou de plusieurs poux de mer, comprenant les étapes consistant à
a) déterminer le niveau d'expression du gène de catalase selon SEQ ID NO: 1 ou d'un variant ou fragment de celui-ci dans un échantillon biologique comprenant de l'ADN ou de l'ARN d'un ou de plusieurs poux de mer, où ledit variant ou fragment de SEQ ID NO: 1 est le gène de catalase selon la revendication 1 ;
b) comparer le niveau d'expression de la catalase déterminé à (i) un étalon contrôle ou (ii) l'expression du gène de catalase ou d'un variant ou fragment de celui-ci dans un échantillon contrôle ;
c) déterminer la différence de niveau d'expression dans l'échantillon par comparaison à l'étalon contrôle ou l'échantillon contrôle ; et
d) caractériser la sensibilité au peroxyde d'hydrogène du ou de plusieurs poux de mer, où le un ou plusieurs poux de mer devant être testés sont résistants s'ils présentent un niveau d'expression plus élevé par comparaison à l'étalon contrôle ou à l'échantillon contrôle.

10. Procédé selon la revendication 9, comprenant les étapes consistant à
- collecter des poux de mer à partir d'un poisson infesté ou d'échantillons d'eau ;
- isoler l'ADN ou l'ARN à partir des poux de mer collectés ;
- mettre à disposition une paire d'amorces de PCR spécifiques au gène de catalase de SEQ ID NO: 1, SEQ ID NO: 13 ou SEQ ID NO: 14, respectivement ;
- réaliser une PCR sur l'ADN ou l'ARN isolé en utilisant la paire d'amorces de PCR ;
- déterminer le niveau d'expression du gène de catalase en comparant le niveau d'expression de la catalase avec le niveau d'un étalon contrôle ou d'un échantillon contrôle de l'expression du gène de catalase.

11. Sonde oligonucléotidique comprenant une séquence de nucléotides, dans laquelle la séquence consiste en celle sélectionnée dans le groupe de SEQ ID NO: 6, 9 et 12 et des fragments et variants de celle-ci présentant au moins 70 % d'identité de séquence sur toute la longueur de SEQ ID NO: 6, 9 et 12.

12. Sonde oligonucléotidique comprenant une séquence de nucléotides selon la revendication 11, dans laquelle la séquence comprend environ 13 à 25 nucléotides consécutifs.

13. Amorce oligonucléotidique comprenant une séquence de nucléotides, dans laquelle la séquence comprend environ 18-25 nucléotides consécutifs et consiste en celle sélectionnée dans le groupe de SEQ ID NO: 2, 3, 4, 5, 7, 8, 10 et 11, et des fragments et variants de celle-ci présentant au moins 70 % d'identité de séquence sur toute la longueur de SEQ ID NO: 2, 3, 4, 5, 7, 8, 10 et 11.

14. Paire d'amorces oligonucléotidiques, dans laquelle les séquences de ladite paire d'amorces sont sélectionnées dans les groupes consistant en SEQ ID NO: 2 et 3, 4 et 5, 7 et 8, et 10 et 11, et des fragments et variants de celles-ci présentant au moins 70 % d'identité de séquence sur toute la longueur de SEQ ID NO: 2 et 3, 4 et 5, 7 et 8, et 10 et 11.

15. Kit pour la détection d'une résistance au peroxyde d'hydrogène (H₂O₂) chez des crustacés, comprenant au moins une sonde, amorce et/ou paire d'amorces oligonucléotidique(s) selon les revendications 11 à 14.

16. Utilisation *in vitro* d'une ou de plusieurs séquence(s) oligonucléotidique(s) isolée(s) comprenant au moins 8 nucléotides contigus de la séquence SEQ ID NO: 1, SEQ ID NO: 13 ou SEQ ID NO: 14, ou d'un oligonucléotide complémentaire de SEQ ID NO: 1, SEQ ID NO: 13 ou SEQ ID NO: 14, respectivement, pour la détermination d'une résistance au peroxyde d'hydrogène chez des poux de mer sélectionnés dans le groupe consistant en *Lepeophtheirus salmonis, Caligus clemensei*, *Caligus elongatus*, et *Caligus rogercresseyi.*


### SEQUENCE LISTING

<110> PatoGen Analyse AS
<120> Method for detecting H2O2 resistance in sea lice
<130> P23605PC00
<150> US62019911
   <151> 2014-07-02
<160> 22
<170> PatentIn version 3.5
<210> 1
   <211> 2007
   <212> DNA
   <213> Lepeoptheirus salmonis
<400> 1
   actttagttt acaattcaaa tccataccgt ataagaacct tcctcgtact acaacatccg 60
   tactagctga ttctaaggaa aattcaaaga tagaaaggag atattttact tgaatatttt 120
   aaacaaagaa aaaggtaagt aggatcatag tttattcaac aaaattagtg taaatatcaa 180
   aatgcaaaga caggcactat ggaaaaatca acacaatctc aaagtttagt ttgttaaagt 240
   gtaatctgga tactacgtct aagaacaata aatatgcctc ttcgtgattg tgccacagaa 300
   caacttgcca actattcaaa gtcgataaag aaagaagacc ttctcacatc cagtcacgga 360
   atcccactgg gtgataaaaa taacatagtt accgttggca gaagaggtcc ttctttgatt 420
   caagatgttg catttatgga cgaaatggcc cattttgtac gtgagcgtat tcccgagaga 480
   gtagttcatg caaaaggagc tggagctttt ggttacttgg aaataactca tgacattaca 540
   aagtactgca aggctgaaat atttgataca attggaaaga gaacagatct tgccgtaagg 600
   ttcagtacag ttggaggtga atctggatct gctgatactg agagagatcc aagaggattt 660
   gccgtcaaat tttatacgaa ggagggtaat tgggacttgg ttggaaacaa tactcctatt 720
   ttttttattc gtgatcccat tctttttgag agttttattc attcacaaaa aagaaatcct 780
   gtaacacatt tgaaggatcc agatatgttt tgggactttg taactctgag gcctgaaaca 840
   acacatcaaa tgtctattct attctcagac agaggaacac cagatggata tagacatatg 900
   aacggctatg gatctcatac ctttaaatta gttaataaaa agaacgaggc tgtatattgt 960
   aagtttcact ttaaaactaa ccaaggtata aagaatcttt caagtcaaga ggctgcaaat 1020
   atggccagag atgatcctga ttactctatt cgcgatttat ataatgctat tgaccaagaa 1080
   aactttcctt cctataccat gcacattcaa gtgatgacta tggatgaagc agacaccata 1140
   gattttgatc cttttgattt gacaaaggtt tggtctcata aaaccttccc actcattgaa 1200
   gtcggacaac tagtcctcaa tagaaatcca tccaattact ttgctgaggt tgaacaaatt 1260
   gcttttagtc cagggaactt gatccctgga attgaagcaa gccctgacaa aatgttgcaa 1320
   ggtcggatat taacctatag tgatgctcac cgctatcgta tttctggtga agtgggacgc 1380
   catgattcct ctgaggaaga taatttttat caagtcggag tgttttatag ggatgtttta 1440
   aacgaagaac aaaagaataa cttggtagac aacattgcag taaatttagt ccatgcacaa 1500
   gaaaaattcc aagtgaaagc catcaaacaa ttcgggcaat gtgatcctga ttatgggaaa 1560
   agacttgaga ctagattggc ttattataaa aataaatgat aaattttgta acataagcat 1620
   gtattacacc ccaaaaaatt gttcccaccc ttaattaaaa aatatatata tatataccaa 1680
   aatattattt tttttcatgc atatgataaa aaaaaatact tgctcacaag acaaatggta 1740
   atgaaacttg tgacaatttc cattaaatac cgttgagata tgagcatcca catcactctg 1800
   actatttgcc aaactgacaa aatcactcgg catttatgta ggggtttttg tttatatctg 1860
   cttggcaaca gtccgctcag tggggatcta tttttgttgc cagaagcaat ttcattcgtt 1920
   tatatgatta aataaacata caactcaaaa tctatagtca aatattattt cactagatta 1980
   tattttgctt tatgctgtag tattaag 2007
<210> 2
   <211> 20
   <212> DNA
   <213> Lepeophtheirus salmonis
<400> 2
   ccacagaaca acttgccaac 20
<210> 3
   <211> 20
   <212> DNA
   <213> Lepeophtheirus salmonis
<400> 3
   gccatttcgt ccataaatgc 20
<210> 4
   <211> 21
   <212> DNA
   <213> Lepeophtheirus salmonis
<400> 4
   tcacatccag tcacggaatc c 21
<210> 5
   <211> 23
   <212> DNA
   <213> Lepeophtheirus salmonis
<400> 5
   acctcttctg ccaacggtaa cta 23
<210> 6
   <211> 16
   <212> DNA
   <213> Lepeophtheirus salmonis
<400> 6
   actgggtgat aaaaat 16
<210> 7
   <211> 24
   <212> DNA
   <213> Lepeophtheirus salmonis
<400> 7
   gcctgaaaca acacatcaaa tgtc 24
<210> 8
   <211> 26
   <212> DNA
   <213> Lepeophtheirus salmonis
<400> 8
   gccgttcata tgtctatatc catctg 26
<210> 9
   <211> 20
   <212> DNA
   <213> Lepeophtheirus salmonis
<400> 9
   tctattctca gacagaggaa 20
<210> 10
   <211> 22
   <212> DNA
   <213> Lepeophtheirus salmonis
<400> 10
   accgttgaga tatgagcatc ca 22
<210> 11
   <211> 22
   <212> DNA
   <213> Lepeophtheirus salmonis
<400> 11
   tgccgagtga ttttgtcagt tt 22
<210> 12
   <211> 18
   <212> DNA
   <213> Lepeophtheirus salmonis
<400> 12
   atcactctga ctatttgc 18
<210> 13
   <211> 1663
   <212> DNA
   <213> Caligus clemensi
<400> 13
   gggggggctt acttcacttg tgtctgacct gtgaatttaa gaagaggaaa atatatataa 60
   ttcatctcca tcatgtccgt tcgtgatcga gcctccgagc aactggcaga ttatgttaaa 120
   acgattaagg gcgctgatcg cctaacatca agtcatggga tccctcttgg cgacaagacc 180
   aacattgtta ccgtaggtaa gagaggccct gctttgcttc aagacgtggc tttcatcgat 240
   gaaatggcgc actttgtccg agaacgcatt cccgagcgcg tggttcatgc caagggtgca 300
   ggggccttcg ggtactttga ggtgacccac gacatcacga aatactccaa agcaggtatc 360
   ttctctgaaa tcggaaagag aacccctttg gctgtaaggt tcagtactgt tgggggcgag 420
   tccggatctg cggatagtgc ccgggatcct aggggctttg cggttaagtt ctacaccaag 480
   gatggaaact gggatttggt tgggaacaac acgcccattt tctttatccg agatcccagt 540
   ctctttagta acttcattca cacccaaaaa aggaatccag tcactcattt gagggaccct 600
   gatatggtct gggactctgt gacccttcgg ccagagacca gccatcaaat atccttcctc 660
   tactctgaca ggggaacccc tgatggatat cgacacatga atggctacgg ctcacataca 720
   tttaaactcg tgaatcaaaa ggacgaggct gtatattgca agttccatat gaaaaccaac 780
   caaggaatca agaatctctc cagcaaggag gctggagact tatcccgtga tgatcctgac 840
   tatgccatcc gtgacttata caactctatc gccggggaaa attatccctc gtacaccatg 900
   cacatccagg tcatgactga ggaacaagct gataacttgg agtctgatcc ctttgacctg 960
   actaaggttt ggtctcataa agccttccct ctcatcgaag tgggacaatt ggtccttaac 1020
   aagaacccag acaactactt ctcagaagtg gagcagattg cctttagccc tgggaacctt 1080
   gtacctggaa tagaacccag ccctgacaaa atgcttcaag gtcgcattct gtcttatagt 1140
   gatgctcatc gttatcgcct tggagcgaat tacactcatc ttaacgttaa ttcaccatac 1200
   aataaggcct gcaattacca acgagatggt cccatgtgca tgagtaatca agggaagggc 1260
   ccaaattact atcccaactc cttttggggg cccagtggat cccaggataa atacggagag 1320
   cacactttct ttgcctcggg cgaagctaaa agacacgaat ccgctcatga ggacaacttt 1380
   tctcaagttg gagagtttta ccgagatatc ttgaatgaag agcaaaagag caacttggtg 1440
   gacaacattg ctagtgattt aattcatgca caagagcact tccaggagaa ggccattaag 1500
   cagttttgtc aatgcgatcc cgactacggc aaaagactgg aagcaaggct ggctctctac 1560
   aaaaataaaa aagtctaagg ggagttccgc tggaaaataa tattcatatt tatatctatg 1620
   atgtatgtac atatttactt aaataaaaaa gcaatgaaat atc 1663
<210> 14
   <211> 3198
   <212> DNA
   <213> Caligus rogercresseiy
<400> 14
   aaaacccatt cttttttctc tgtataaata ttgtggattt attagtaagt gggtttagtt 60
   gataataatc cagactacac gacttaagaa aaaggttcaa catgtctgtt cgtgatagag 120
   cgactgagca gcttgctgat tactccaaga caatcaagaa tattgagcgt ctaactacaa 180
   gtcatgggat ccccatcggt gataaaacca atataatcac catcggcaag cggggcccat 240
   ccctgctcca ggacgtggcc ttcattgacg aaatagcgca ttttgtccgt gagcgcattc 300
   ctgagcgggt cgttcatgcc aagggtgcgg gcgcattcgg atacttcgaa gtgacccatg 360
   acatatcaaa atacaccaag gccgacatat tcaagagcat tggaaagcga acccccgtgg 420
   ccgttcgctt tagcacagtg ggcggggagt ctggctcagc agacagcgtg agggacccca 480
   ggggcttcgc cgtcaagttt tacacggacg acggcaactg ggacttggtg gggaacaata 540
   cccccatctt ttttattcgg gatcctagcc tctttggaaa ctttatccat tctcaaaaga 600
   ggaatcctgt gacacacttg agggaccccg acatggtatg ggactttgtc acactccgac 660
   cagagacaag tcatcaaata tccatccttt attcggaacg ggggacccct gatggatatc 720
   gacacatgaa tggctatgga tcacatacct ttaaactggt taacaacaaa aacgaaattg 780
   tgtattgtaa gtttcacttg aagacaaatc aagggataaa gaatctctca agcagccgag 840
   cggccgagtt aacaaaggat gatcccgact attctattcg ggacttgtac aattccatcg 900
   ccgaggataa ttatccctct tacaccatgc acatccaagt gatgacagat gaggaggccg 960
   ataaactgga gtttgatccc tttgatttaa ccaaagtctg gtctcacaag gactttcctc 1020
   tcattgaagt gggtcagctc gtcctcgata agaacccaaa taattacttt gctgaggtgg 1080
   agcaaatcgc tttcagtccc ggaaatctgg ttccagggat tgagccaagt cctgataaaa 1140
   tgcttcaggg tcgcatttta tcctatagtg atgctcatcg ctatcgtctg ggtgccaatt 1200
   atgctcaaat tcaagttaat tcctctttta ataagtcatg taactaccag cgagatggtc 1260
   atatgtgcat gaacaatcaa aaccgaggtc caaattactt tcccaactca ttttcgggtc 1320
   ctaatggatc caaggataaa tatggagagc attcctttgt agcctccgga gagataaaga 1380
   gacacgattc cgcggatgaa gataacttta atcaggtggg attattctac agggacgtct 1440
   tgagtgaaga gcaaaaagac aatttagtgg acaacattgc agagcatctt gttcaagcgc 1500
   aggagatatt ccaggaaaag gccatcaaac aatttggaca atgtgatgcg gactacggga 1560
   gaagattgga ggccagactt aattactaca agaataaagc ctagttttgt aaaaaaatct 1620
   atttatttga attattttgt atgctgcaaa aatttgacat accttaaaac atcttactta 1680
   atgtatataa ataataaaaa ctctcttcat gaattaaatg atgcttaaag attgatgtaa 1740
   aacttttgtg ggactaatat aatatataac gttatgtaaa tacgatcatg taaaaagaaa 1800
   ataaattgaa atatcataat atattaaaat atatattttt tgaagttgcg ataatacaaa 1860
   cgatgatttt gtgcaagact acttcagtct cttaggaagc gggattggaa ttcatttagt 1920
   tatttgtctc tagggttgga gggtgcaatt catttttgat attatatttc ttgatatata 1980
   tatatagact attttttaaa gaaaataaat gaattgaaaa ctgcgaatat accgttttac 2040
   tatcccagat ttgcgcttat cgttgttgtt gaacccggaa ccattaaaaa tcattgtatt 2100
   taatgtgtta aattggttaa taatatggtc cgacttgagg aactaaaaga ataaaaaaat 2160
   gtaaaatatt aattgacttc attacttaat tttgtacttt tttctctttt tctatccttt 2220
   atagtaattg gatatgtaat gaaaaaatgg ccactgtttt ccgcatatat atgttatgtt 2280
   caatacattt atgtaacaaa caataaaata tcaatttagt ttattaataa catacatttg 2340
   tatttaagaa tgtttagtat caggttattc agtcagccta agaatataaa caaaataaaa 2400
   agtttaataa aacgctctat aataaaaaag aaagaatcac tagaatgatg ggcatcacat 2460
   ggttttttgt ttcaaatcct ttcgttttaa aagtttactt ttaacacttc cttcgtgagg 2520
   aaacttaggt ggctgaaaat gttgtgtttt atctttcttt cctatatttg ttttaattaa 2580
   gttatttaag gtgaaatgtt tatttgtact cttttatata taaactggga attttataaa 2640
   aacttaatca ttcgatttca atttatttct ctttcacacg ctgcttcatg atttgtagtt 2700
   taatcacaaa tctcaggatc ctccaatgta cagttttggt gtaaagcact caagctatat 2760
   ctacggcgcc cttccccttc caggagagga gactcgtcct cttccttact aacatcattc 2820
   tcccaacctc ccacacaaac ctactcaatg cctctaacaa gtttttcctt tccctcaggt 2880
   ggacgcactt cttcattcat caccaaaaat ctccttcggt atccgacatt ctccttacat 2940
   taccatgctt aaaggcgaca actatgtcac tacctacaca gacagtgttg aggaggaacc 3000
   ctacaggtct tcttctactt catttgacgg agcctctaat aaggtcacaa ctacctacag 3060
   ttataacatt gtgaaaaatg gatccaacaa tgttaatagt aatgagtcca gctatatcgt 3120
   tcaagagcct tcccaagaac ccagtcataa ttccgcaacg acttacatac tcagaaatcc 3180
   atccacgcag tccaatat 3198
<210> 15
   <211> 24
   <212> DNA
   <213> Caligus rogercresseyi
<400> 15
   aagaggaatc ctgtgacaca cttg 24
<210> 16
   <211> 22
   <212> DNA
   <213> Caligus rogercresseyi
<400> 16
   ctggtcggag tgtgacaaag tc 22
<210> 17
   <211> 16
   <212> DNA
   <213> Caligus rogercresseyi
<400> 17
   accccgacat ggtatg 16
<210> 18
   <211> 19
   <212> DNA
   <213> Caligus clemensi
<400> 18
   gcgcactttg tccgagaac 19
<210> 19
   <211> 19
   <212> DNA
   <213> Caligus clemensi
<400> 19
   ctgcaccctt ggcatgaac 19
<210> 20
   <211> 15
   <212> DNA
   <213> Caligus clemensi
<400> 20
   cattcccgag cgcgt 15
<210> 21
   <211> 1506
   <212> DNA
   <213> Caligus clemensi
<400> 21
   atgtccgttc gtgatcgagc ctccgagcaa ctggcagatt atgttaaaac gattaagggc    60
   gctgatcgcc taacatcaag tcatgggatc cctcttggcg acaagaccaa cattgttacc   120
   gtaggtaaga gaggccctgc tttgcttcaa gacgtggctt tcatcgatga aatggcgcac   180
   tttgtccgag aacgcattcc cgagcgcgtg gttcatgcca agggtgcagg ggccttcggg   240
   tactttgagg tgacccacga catcacgaaa tactccaaag caggtatctt ctctgaaatc   300
   ggaaagagaa cccctttggc tgtaaggttc agtactgttg ggggcgagtc cggatctgcg    360
   gatagtgccc gggatcctag gggctttgcg gttaagttct acaccaagga tggaaactgg   420
   gatttggttg ggaacaacac gcccattttc tttatccgag atcccagtct ctttagtaac   480
   ttcattcaca cccaaaaaag gaatccagtc actcatttga gggaccctga tatggtctgg   540
   gactctgtga cccttcggcc agagaccagc catcaaatat ccttcctcta ctctgacagg   600
   ggaacccctg atggatatcg acacatgaat ggctacggct cacatacatt taaactcgtg  660
   aatcaaaagg acgaggctgt atattgcaag ttccatatga aaaccaacca aggaatcaag   720
   aatctctcca gcaaggaggc tggagactta tcccgtgatg atcctgacta tgccatccgt   780
   gacttataca actctatcgc cggggaaaat tatccctcgt acaccatgca catccaggtc   840
   atgactgagg aacaagctga taacttggag tctgatccct ttgacctgac taaggtttgg   900
   tctcataaag ccttccctct catcgaagtg ggacaattgg tccttaacaa gaacccagac   960
   aactacttct cagaagtgga gcagattgcc tttagccctg ggaaccttgt acctggaata  1020
   gaacccagcc ctgacaaaat gcttcaaggt cgcattctgt cttatagtga tgctcatcgt  1080
   tatcgccttg gagcgaatta cactcatctt aacgttaatt caccatacaa taaggcctgc  1140
   aattaccaac gagatggtcc catgtgcatg agtaatcaag ggaagggccc aaattactat  1200
   cccaactcct tttgggggcc cagtggatcc caggataaat acggagagca cactttcttt  1260
   gcctcgggcg aagctaaaag acacgaatcc gctcatgagg acaacttttc tcaagttgga  1320
   gagttttacc gagatatctt gaatgaagag caaaagagca acttggtgga caacattgct  1380
   agtgatttaa ttcatgcaca agagcacttc caggagaagg ccattaagca gttttgtcaa  1440
   tgcgatcccg actacggcaa aagactggaa gcaaggctgg ctctctacaa aaataaaaaa  1500
   gtctaa                                                             1506
<210> 22
   <211> 2109
   <212> DNA
   <213> Octupus vulgaris
<400> 22 SEQUENCE LISTING
<110> PatoGen Analyse AS
<120> Method for detecting H2O2 resistance in sea lice
<130> P23605PC00
<150> US62019911
   <151> 2014-07-02
<160> 22
<170> PatentIn version 3.5
<210> 1
   <211> 2007
   <212> DNA
   <213> Lepeoptheirus salmonis
<400> 1
   actttagttt acaattcaaa tccataccgt ataagaacct tcctcgtact acaacatccg        60
   tactagctga ttctaaggaa aattcaaaga tagaaaggag atattttact tgaatatttt       120
   aaacaaagaa aaaggtaagt aggatcatag tttattcaac aaaattagtg taaatatcaa       180
   aatgcaaaga caggcactat ggaaaaatca acacaatctc aaagtttagt ttgttaaagt       240
   gtaatctgga tactacgtct aagaacaata aatatgcctc ttcgtgattg tgccacagaa       300
   caacttgcca actattcaaa gtcgataaag aaagaagacc ttctcacatc cagtcacgga       360
   atcccactgg gtgataaaaa taacatagtt accgttggca gaagaggtcc ttctttgatt       420
   caagatgttg catttatgga cgaaatggcc cattttgtac gtgagcgtat tcccgagaga       480
   gtagttcatg caaaaggagc tggagctttt ggttacttgg aaataactca tgacattaca       540
   aagtactgca aggctgaaat atttgataca attggaaaga gaacagatct tgccgtaagg       600
   ttcagtacag ttggaggtga atctggatct gctgatactg agagagatcc aagaggattt       660
   gccgtcaaat tttatacgaa ggagggtaat tgggacttgg ttggaaacaa tactcctatt       720
   ttttttattc gtgatcccat tctttttgag agttttattc attcacaaaa aagaaatcct       780
   gtaacacatt tgaaggatcc agatatgttt tgggactttg taactctgag gcctgaaaca       840
   acacatcaaa tgtctattct attctcagac agaggaacac cagatggata tagacatatg       900
   aacggctatg gatctcatac ctttaaatta gttaataaaa agaacgaggc tgtatattgt       960
   aagtttcact ttaaaactaa ccaaggtata aagaatcttt caagtcaaga ggctgcaaat      1020
   atggccagag atgatcctga ttactctatt cgcgatttat ataatgctat tgaccaagaa      1080
   aactttcctt cctataccat gcacattcaa gtgatgacta tggatgaagc agacaccata      1140
   gattttgatc cttttgattt gacaaaggtt tggtctcata aaaccttccc actcattgaa      1200
   gtcggacaac tagtcctcaa tagaaatcca tccaattact ttgctgaggt tgaacaaatt      1260
   gcttttagtc cagggaactt gatccctgga attgaagcaa gccctgacaa aatgttgcaa     1320
   ggtcggatat taacctatag tgatgctcac cgctatcgta tttctggtga agtgggacgc     1380
   catgattcct ctgaggaaga taatttttat caagtcggag tgttttatag ggatgtttta     1440
   aacgaagaac aaaagaataa cttggtagac aacattgcag taaatttagt ccatgcacaa     1500
   gaaaaattcc aagtgaaagc catcaaacaa ttcgggcaat gtgatcctga ttatgggaaa     1560
   agacttgaga ctagattggc ttattataaa aataaatgat aaattttgta acataagcat     1620
   gtattacacc ccaaaaaatt gttcccaccc ttaattaaaa aatatatata tatataccaa     1680
   aatattattt tttttcatgc atatgataaa aaaaaatact tgctcacaag acaaatggta     1740
   atgaaacttg tgacaatttc cattaaatac cgttgagata tgagcatcca catcactctg     1800
   actatttgcc aaactgacaa aatcactcgg catttatgta ggggtttttg tttatatctg     1860
   cttggcaaca gtccgctcag tggggatcta tttttgttgc cagaagcaat ttcattcgtt     1920
   tatatgatta aataaacata caactcaaaa tctatagtca aatattattt cactagatta     1980
   tattttgctt tatgctgtag tattaag                                         2007
<210> 2
   <211> 20
   <212> DNA
   <213> Lepeophtheirus salmonis
<400> 2
   ccacagaaca acttgccaac 20
<210> 3
   <211> 20
   <212> DNA
   <213> Lepeophtheirus salmonis
<400> 3
   gccatttcgt ccataaatgc 20
<210> 4
   <211> 21
   <212> DNA
   <213> Lepeophtheirus salmonis
<400> 4
   tcacatccag tcacggaatc c 21
<210> 5
   <211> 23
   <212> DNA
   <213> Lepeophtheirus salmonis
<400> 5
   acctcttctg ccaacggtaa cta 23
<210> 6
   <211> 16
   <212> DNA
   <213> Lepeophtheirus salmonis
<400> 6
   actgggtgat aaaaat 16
<210> 7
   <211> 24
   <212> DNA
   <213> Lepeophtheirus salmonis
<400> 7
   gcctgaaaca acacatcaaa tgtc 24
<210> 8
   <211> 26
   <212> DNA
   <213> Lepeophtheirus salmonis
<400> 8
   gccgttcata tgtctatatc catctg 26
<210> 9
   <211> 20
   <212> DNA
   <213> Lepeophtheirus salmonis
<400> 9
   tctattctca gacagaggaa 20
<210> 10
   <211> 22
   <212> DNA
   <213> Lepeophtheirus salmonis
<400> 10
   accgttgaga tatgagcatc ca 22
<210> 11
   <211> 22
   <212> DNA
   <213> Lepeophtheirus salmonis
<400> 11
   tgccgagtga ttttgtcagt tt 22
<210> 12
   <211> 18
   <212> DNA
   <213> Lepeophtheirus salmonis
<400> 12
   atcactctga ctatttgc 18
<210> 13
   <211> 1663
   <212> DNA
   <213> Caligus clemensi
<400> 13
   gggggggctt acttcacttg tgtctgacct gtgaatttaa gaagaggaaa atatatataa      60
   ttcatctcca tcatgtccgt tcgtgatcga gcctccgagc aactggcaga ttatgttaaa     120
   acgattaagg gcgctgatcg cctaacatca agtcatggga tccctcttgg cgacaagacc     180
   aacattgtta ccgtaggtaa gagaggccct gctttgcttc aagacgtggc tttcatcgat     240
   gaaatggcgc actttgtccg agaacgcatt cccgagcgcg tggttcatgc caagggtgca     300
   ggggccttcg ggtactttga ggtgacccac gacatcacga aatactccaa agcaggtatc     360
   ttctctgaaa tcggaaagag aacccctttg gctgtaaggt tcagtactgt tgggggcgag      420
   tccggatctg cggatagtgc ccgggatcct aggggctttg cggttaagtt ctacaccaag     480
   gatggaaact gggatttggt tgggaacaac acgcccattt tctttatccg agatcccagt     540
   ctctttagta acttcattca cacccaaaaa aggaatccag tcactcattt gagggaccct     600
   gatatggtct gggactctgt gacccttcgg ccagagacca gccatcaaat atccttcctc     660
   tactctgaca ggggaacccc tgatggatat cgacacatga atggctacgg ctcacataca     720
   tttaaactcg tgaatcaaaa ggacgaggct gtatattgca agttccatat gaaaaccaac     780
   caaggaatca agaatctctc cagcaaggag gctggagact tatcccgtga tgatcctgac     840
   tatgccatcc gtgacttata caactctatc gccggggaaa attatccctc gtacaccatg     900
   cacatccagg tcatgactga ggaacaagct gataacttgg agtctgatcc ctttgacctg     960
   actaaggttt ggtctcataa agccttccct ctcatcgaag tgggacaatt ggtccttaac    1020
   aagaacccag acaactactt ctcagaagtg gagcagattg cctttagccc tgggaacctt    1080
   gtacctggaa tagaacccag ccctgacaaa atgcttcaag gtcgcattct gtcttatagt    1140
   gatgctcatc gttatcgcct tggagcgaat tacactcatc ttaacgttaa ttcaccatac    1200
   aataaggcct gcaattacca acgagatggt cccatgtgca tgagtaatca agggaagggc    1260
   ccaaattact atcccaactc cttttggggg cccagtggat cccaggataa atacggagag    1320
   cacactttct ttgcctcggg cgaagctaaa agacacgaat ccgctcatga ggacaacttt    1380
   tctcaagttg gagagtttta ccgagatatc ttgaatgaag agcaaaagag caacttggtg    1440
   gacaacattg ctagtgattt aattcatgca caagagcact tccaggagaa ggccattaag    1500
   cagttttgtc aatgcgatcc cgactacggc aaaagactgg aagcaaggct ggctctctac    1560
   aaaaataaaa aagtctaagg ggagttccgc tggaaaataa tattcatatt tatatctatg    1620
   atgtatgtac atatttactt aaataaaaaa gcaatgaaat atc                      1663
<210> 14
   <211> 3198
   <212> DNA
   <213> Caligus rogercresseiy
<400> 14
   aaaacccatt cttttttctc tgtataaata ttgtggattt attagtaagt gggtttagtt 60
   gataataatc cagactacac gacttaagaa aaaggttcaa catgtctgtt cgtgatagag 120
   cgactgagca gcttgctgat tactccaaga caatcaagaa tattgagcgt ctaactacaa 180
   gtcatgggat ccccatcggt gataaaacca atataatcac catcggcaag cggggcccat 240
   ccctgctcca ggacgtggcc ttcattgacg aaatagcgca ttttgtccgt gagcgcattc 300
   ctgagcgggt cgttcatgcc aagggtgcgg gcgcattcgg atacttcgaa gtgacccatg 360
   acatatcaaa atacaccaag gccgacatat tcaagagcat tggaaagcga acccccgtgg 420
   ccgttcgctt tagcacagtg ggcggggagt ctggctcagc agacagcgtg agggacccca 480
   ggggcttcgc cgtcaagttt tacacggacg acggcaactg ggacttggtg gggaacaata 540
   cccccatctt ttttattcgg gatcctagcc tctttggaaa ctttatccat tctcaaaaga 600
   ggaatcctgt gacacacttg agggaccccg acatggtatg ggactttgtc acactccgac 660
   cagagacaag tcatcaaata tccatccttt attcggaacg ggggacccct gatggatatc 720
   gacacatgaa tggctatgga tcacatacct ttaaactggt taacaacaaa aacgaaattg 780
   tgtattgtaa gtttcacttg aagacaaatc aagggataaa gaatctctca agcagccgag 840
   cggccgagtt aacaaaggat gatcccgact attctattcg ggacttgtac aattccatcg 900
   ccgaggataa ttatccctct tacaccatgc acatccaagt gatgacagat gaggaggccg 960
   ataaactgga gtttgatccc tttgatttaa ccaaagtctg gtctcacaag gactttcctc 1020
   tcattgaagt gggtcagctc gtcctcgata agaacccaaa taattacttt gctgaggtgg 1080
   agcaaatcgc tttcagtccc ggaaatctgg ttccagggat tgagccaagt cctgataaaa 1140
   tgcttcaggg tcgcatttta tcctatagtg atgctcatcg ctatcgtctg ggtgccaatt 1200
   atgctcaaat tcaagttaat tcctctttta ataagtcatg taactaccag cgagatggtc 1260
   atatgtgcat gaacaatcaa aaccgaggtc caaattactt tcccaactca ttttcgggtc 1320
   ctaatggatc caaggataaa tatggagagc attcctttgt agcctccgga gagataaaga 1380
   gacacgattc cgcggatgaa gataacttta atcaggtggg attattctac agggacgtct 1440
   tgagtgaaga gcaaaaagac aatttagtgg acaacattgc agagcatctt gttcaagcgc 1500
   aggagatatt ccaggaaaag gccatcaaac aatttggaca atgtgatgcg gactacggga 1560
   gaagattgga ggccagactt aattactaca agaataaagc ctagttttgt aaaaaaatct 1620
   atttatttga attattttgt atgctgcaaa aatttgacat accttaaaac atcttactta 1680
   atgtatataa ataataaaaa ctctcttcat gaattaaatg atgcttaaag attgatgtaa 1740
   aacttttgtg ggactaatat aatatataac gttatgtaaa tacgatcatg taaaaagaaa 1800
   ataaattgaa atatcataat atattaaaat atatattttt tgaagttgcg ataatacaaa 1860
   cgatgatttt gtgcaagact acttcagtct cttaggaagc gggattggaa ttcatttagt 1920
   tatttgtctc tagggttgga gggtgcaatt catttttgat attatatttc ttgatatata 1980
   tatatagact attttttaaa gaaaataaat gaattgaaaa ctgcgaatat accgttttac 2040
   tatcccagat ttgcgcttat cgttgttgtt gaacccggaa ccattaaaaa tcattgtatt 2100
   taatgtgtta aattggttaa taatatggtc cgacttgagg aactaaaaga ataaaaaaat 2160
   gtaaaatatt aattgacttc attacttaat tttgtacttt tttctctttt tctatccttt 2220
   atagtaattg gatatgtaat gaaaaaatgg ccactgtttt ccgcatatat atgttatgtt 2280
   caatacattt atgtaacaaa caataaaata tcaatttagt ttattaataa catacatttg 2340
   tatttaagaa tgtttagtat caggttattc agtcagccta agaatataaa caaaataaaa 2400
   agtttaataa aacgctctat aataaaaaag aaagaatcac tagaatgatg ggcatcacat 2460
   ggttttttgt ttcaaatcct ttcgttttaa aagtttactt ttaacacttc cttcgtgagg 2520
   aaacttaggt ggctgaaaat gttgtgtttt atctttcttt cctatatttg ttttaattaa 2580
   gttatttaag gtgaaatgtt tatttgtact cttttatata taaactggga attttataaa 2640
   aacttaatca ttcgatttca atttatttct ctttcacacg ctgcttcatg atttgtagtt 2700
   taatcacaaa tctcaggatc ctccaatgta cagttttggt gtaaagcact caagctatat 2760
   ctacggcgcc cttccccttc caggagagga gactcgtcct cttccttact aacatcattc 2820
   tcccaacctc ccacacaaac ctactcaatg cctctaacaa gtttttcctt tccctcaggt 2880
   ggacgcactt cttcattcat caccaaaaat ctccttcggt atccgacatt ctccttacat 2940
   taccatgctt aaaggcgaca actatgtcac tacctacaca gacagtgttg aggaggaacc 3000
   ctacaggtct tcttctactt catttgacgg agcctctaat aaggtcacaa ctacctacag 3060
   ttataacatt gtgaaaaatg gatccaacaa tgttaatagt aatgagtcca gctatatcgt 3120
   tcaagagcct tcccaagaac ccagtcataa ttccgcaacg acttacatac tcagaaatcc 3180
   atccacgcag tccaatat 3198
<210> 15
   <211> 24
   <212> DNA
   <213> Caligus rogercresseyi
<400> 15
   aagaggaatc ctgtgacaca cttg 24
<210> 16
   <211> 22
   <212> DNA
   <213> Caligus rogercresseyi
<400> 16
   ctggtcggag tgtgacaaag tc 22
<210> 17
   <211> 16
   <212> DNA
   <213> Caligus rogercresseyi
<400> 17
   accccgacat ggtatg 16
<210> 18
   <211> 19
   <212> DNA
   <213> Caligus clemensi
<400> 18
   gcgcactttg tccgagaac 19
<210> 19
   <211> 19
   <212> DNA
   <213> Caligus clemensi
<400> 19
   ctgcaccctt ggcatgaac 19
<210> 20
   <211> 15
   <212> DNA
   <213> Caligus clemensi
<400> 20
   cattcccgag cgcgt 15
<210> 21
   <211> 1506
   <212> DNA
   <213> Caligus clemensi
<400> 21
   atgtccgttc gtgatcgagc ctccgagcaa ctggcagatt atgttaaaac gattaagggc        60
   gctgatcgcc taacatcaag tcatgggatc cctcttggcg acaagaccaa cattgttacc       120
   gtaggtaaga gaggccctgc tttgcttcaa gacgtggctt tcatcgatga aatggcgcac       180
   tttgtccgag aacgcattcc cgagcgcgtg gttcatgcca agggtgcagg ggccttcggg       240
   tactttgagg tgacccacga catcacgaaa tactccaaag caggtatctt ctctgaaatc       300
   ggaaagagaa cccctttggc tgtaaggttc agtactgttg ggggcgagtc cggatctgcg        360
   gatagtgccc gggatcctag gggctttgcg gttaagttct acaccaagga tggaaactgg       420
   gatttggttg ggaacaacac gcccattttc tttatccgag atcccagtct ctttagtaac      480
   ttcattcaca cccaaaaaag gaatccagtc actcatttga gggaccctga tatggtctgg      540
   gactctgtga cccttcggcc agagaccagc catcaaatat ccttcctcta ctctgacagg      600
   ggaacccctg atggatatcg acacatgaat ggctacggct cacatacatt taaactcgtg      660
   aatcaaaagg acgaggctgt atattgcaag ttccatatga aaaccaacca aggaatcaag      720
   aatctctcca gcaaggaggc tggagactta tcccgtgatg atcctgacta tgccatccgt      780
   gacttataca actctatcgc cggggaaaat tatccctcgt acaccatgca catccaggtc      840
   atgactgagg aacaagctga taacttggag tctgatccct ttgacctgac taaggtttgg      900
   tctcataaag ccttccctct catcgaagtg ggacaattgg tccttaacaa gaacccagac      960
   aactacttct cagaagtgga gcagattgcc tttagccctg ggaaccttgt acctggaata     1020
   gaacccagcc ctgacaaaat gcttcaaggt cgcattctgt cttatagtga tgctcatcgt     1080
   tatcgccttg gagcgaatta cactcatctt aacgttaatt caccatacaa taaggcctgc     1140
   aattaccaac gagatggtcc catgtgcatg agtaatcaag ggaagggccc aaattactat     1200
   cccaactcct tttgggggcc cagtggatcc caggataaat acggagagca cactttcttt     1260
   gcctcgggcg aagctaaaag acacgaatcc gctcatgagg acaacttttc tcaagttgga     1320
   gagttttacc gagatatctt gaatgaagag caaaagagca acttggtgga caacattgct     1380
   agtgatttaa ttcatgcaca agagcacttc caggagaagg ccattaagca gttttgtcaa     1440
   tgcgatcccg actacggcaa aagactggaa gcaaggctgg ctctctacaa aaataaaaaa     1500
   gtctaa                                                                1506
<210> 22
   <211> 2109
   <212> DNA
   <213> Octupus vulgaris
<400> 22
   gtcatctgcc gttatcagtg ttctagttac gtcccctgat ctcctaatgt ctttttattt        60
   actatctgcg aagatataag cttcagttag cagctactgt cccataacaa tcaagcgttc       120
   atcgaacttt caatcatggc caaccgagac aaagttagcg accaacttaa gcaatacaag       180
   aaagatcatc cgaaaagtga gacattgaca actggcaccg gtgctcctgt tgggaataaa       240
   aatgcttctt tgactgttgg cccaagaggt ccaattttgt tacaagactt tgtgtttact       300
   gatgaaatgg cacatttcaa cagagaaaga attcctgaaa gagttgttca tgcaaaagga       360
   gctggagctt ttggttactt tgaaactact catgatatta agaaatattg caaagctgct       420
   cctttccaga caattggtaa aagaacaaaa gttggaatac ggttttcaac agtaggtggt       480
   gaaagtggat cagctgacac agcccgggat ccccgaggat ttgcagttaa attttacact       540
   gaagatggaa attgggatct tgttggcaac aatactccta ttttcttcat cagagatcca     600
   attctgtttc ccagttttat acacacacag aaaagaaacc ccaaaacaca ttgtaaggat     660
   ccagatatgt tttgggactt cattactctg cggcctgaaa ctactcatca agtatgtttt     720
   ctgttctctg atcgtggcac accagatggc tatcgcagaa tgaatggtta tggaagtcat     780
   acatttaaga tggtcaacca aaataatgaa gctgtctatt gtaaatttca ctttaaaaca     840
   aatcaaggta taaagaattt aatggctgat gaagcagcca agctgagtgc aagtgaccca     900
   gattatgcca tcagagactt acacaatgca attgagaatg gtaatttccc atcctggact     960
   ctgtacattc aagtgatgac atttaaagaa gcagaaaatt tccgatggaa tccatttgat    1020
   ttaactaaaa tctggcctca tagtgaatat ccactaatac ctgttggacg catggttctt    1080
   gacaccaatc ccaaaaatta ttttgctgag gttgaacaga ttgctttctc acctgcacat    1140
   atgattccag gaattgaacc aagcccagat aaaatgttac agggtcgcct gtattcctac    1200
   tctgacactc atcggcatcg cttaggttct aactatctcc agctaccagt caactgtcct    1260
   ttcagaagcc gtttagccaa ctatcaaagg gacggccctc agtgtgtaac tgacaaccaa    1320
   ggaggagctc ctaattatta ccctaacagc ttttctggtc ctgttgacga tattcaatac    1380
   gtgaagaagc cattttcagc ttctggagat gttcagcgtt acaatactga agacactgac    1440
   aactattctc aagttggaac attttggctg aagatattgt caccagctga acgtgaaaga    1500
   cttgtggaga acactgctaa tcatttgatt gatgcccaag agtttattca aaagcgtgct    1560
   gtgcacaatt ttggacgagt gaattcagaa tttggccata agttacaggt gaaactggac    1620
   agtttgaagt cttcaaaaaa tgtgagttcc caattaattg tatcaaactt atagaccaaa    1680
   gagaaagaga aaaaggaaaa ggcaaaataa caaaatctat tcaataattc atgatgtcct    1740
   ggctgatcaa aagatgctga aactattgaa ttttaatttc agatatctta ttcttaattg    1800
   gaattagatc ctattgaagc acttaaagtg ggtttcatta gcaatataat gcaaaatatt    1860
   taagaagccc caaatgattg atttaacagt tttgttttta tttcactaaa tatatattca    1920
   tgttttaaaa ctgtgatctg gaactgaata aactacttaa aatacaaaac atgtaatagg    1980
   ctaatataac aaaatcatat tctattagat ataattaatt tcctacaaga tttaaattat    2040
   tattgcgagg aacatgctgc aatactacta tgattatttt taaaaaaaaa aaaaaaaaaa    2100
   aaaaaaaaa                                                            2109